(19) Europäisches Patentamt
European Patent Office
Office européen des brevets

(11) EP 0 641 759 B1

(12) EUROPÄISCHE PATENTSCHRIFT

(45) Veröffentlichungstag und Bekanntmachung des Hinweises auf die Patenterteilung:
**09.12.1998 Patentblatt 1998/50**

(21) Anmeldenummer: **94112886.0**

(22) Anmeldetag: **18.08.1994**

(51) Int. Cl.$^6$: **C07C 57/50**, C07C 59/72, C07C 59/64, C07C 63/64, C07C 63/66, C07C 69/618, C07C 69/734, C07C 69/76, C07C 69/92, C07C 69/94, C07C 51/00

# (54) Verwendung aromatischer Carbonsäurederivate zur Herstellung von pharmazeutischen Präparaten

Use of aromatic carboxylic derivatives for the preparation of drugs

Utilisation de dérivés d'acides aromatiques carboxyliques pour la préparation de médicaments

(84) Benannte Vertragsstaaten:
**AT BE CH DE DK ES FR GB GR IE IT LI LU MC NL PT SE**

(30) Priorität: **02.09.1993 CH 2618/93**
**21.06.1994 CH 1960/94**

(43) Veröffentlichungstag der Anmeldung:
**08.03.1995 Patentblatt 1995/10**

(73) Patentinhaber:
**F. HOFFMANN-LA ROCHE AG**
**4002 Basel (CH)**

(72) Erfinder:
- **Klaus, Michael**
**D-79576 Weil am Rhein (CH)**
- **Mohr, Peter**
**CH-4054 Basel (CH)**

(74) Vertreter:
**Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 002 742**
**EP-A- 0 169 571**
**EP-A- 0 315 071**
**US-A- 4 783 549**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 641 759 B1

Printed by Xerox (UK) Business Services
2.16.6/3.4

## Beschreibung

Die vorliegende Erfindung betrifft **die Verwendung** aromatischer Carbonsäurederivate der Formel

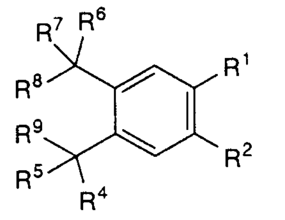

I

worin

$R^1$ einen Rest der Formeln

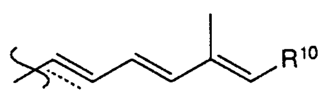

(a)

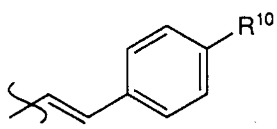

(b)

$R^2$ $C_{2-8}$-Alkanoyl, $C_{2-8}$-Alkyl, $C_{2-8}$-Alkenyl, $C_{2-8}$-Alkinyl oder $-OCH_2R^3$;
$R^3$ Wasserstoff oder $C_{1-6}$-Alkyl, $C_{2-6}$-Alkenyl oder $C_{2-6}$-Alkinyl;
$R^4$ bis $R^9$ unabhängig voneinander Wasserstoff oder $C_{1-5}$-Alkyl bedeuten; oder
$R^8$ und $R^9$ zusammengenommen $(CR^aR^b)_n$, $R^a$ und $R^8$ Wasserstoff oder $C_{1-5}$-Alkyl, n 1, 2 oder 3 und $R^4$ bis $R^7$ dasselbe wie oben bedeuten; oder
$R^8$ und $R^9$ zusammengenommen $(CR^aR^b)_n$, und $R^4$ und $R^6$ zusammengenommen Methylen oder Aethylen, die durch Hydroxy substituiert sein können, und $R^a$, $R^b$, $R^5$, $R^7$ und n dasselbe wie oben bedeuten;
$R^{10}$ Carboxyl, $C_{1-6}$-Alkoxycarbonyl oder mono- oder di-($C_{1-6}$-Alkyl)carbamoyl darstellen, und die punktierte Bindung in der Formel (a) fakultativ ist;

und pharmazeutisch anwendbare Salze von Carbonsäuren der Formel I, **bei der Herstellung von pharmazeutischen Präparaten zur Therapie und Prophylaxe von licht- und altersbedingten Schäden der Haut sowie zur Förderung der Wundheilung.**

**Aromatische Cabonsäure-Derivate sind bereits für die Therapie von Akne und Psoriasis bekannt, so werden in US Patent Nr. 4 783 549 Benzonorbornen-Derivate, in EP-A-315 071 Benzocyclohepten-Derivate und in EP-A-2 742 Stilben-Derivate für diesen Zweck beschrieben.**

Der hier verwendete Ausdruck "$C_{1-6}$", "$C_{2-6}$", "$C_{1-5}$" und "$C_{2-8}$" bezeichnet Gruppen mit 1-6, 2-6, 1-5 und 2-8 Kohlenstoffatomen. Beispiele von Alkylresten $R^2$ und $R^3$ sind vorzugsweise geradkettige Alkylreste wie Aethyl, Propyl, Butyl, Pentyl, Hexyl, Heptyl und Octyl. Beispiele von Alkenylresten $R^2$ und $R^3$ sind vorzugsweise geradkettige Alkenylreste wie Vinyl, 1- und 2-Propenyl, und 2-Butenyl. Beispiele von Alkinylresten $R^2$ und $R^3$ sind Aethinyl, 1- und 2-Propinyl, 1- und 2-Butinyl. Beispiele von $C_{2-8}$-Alkanoylresten sind vorzugsweise geradkettige Alkanoylreste wie Acetyl, Propionyl, Butyryl, Pentanoyl, Hexanoyl, Heptanoyl und Octanoyl.

In einer Ausführungsform betrifft die Erfindung **die Verwendung der** Verbindungen der Formel I, in denen $R^4$ bis $R^7$ unabhängig voneinander Wasserstoff oder $C_{1-5}$-Alkyl und $R^8$ und $R^9$ zusammengenommen $(CR^aR^b)_n$ bedeuten. Von diesen sind diejenigen bevorzugt, in denen $R^4$ bis $R^7$ Methyl und $R^8$ und $R^9$ zusammen Methylen, Aethylen oder Propylen bedeuten.

In einer anderen Ausführungsform betrifft die Erfindung **die Verwendung der** Verbindungen der Formel I, in denen $R^8$ und $R^9$ zusammengenommen $(CR^aR^b)_n$, $R^5$ und $R^7$ Wasserstoff oder $C_{1-5}$-Alkyl und $R^4$ und $R^6$ zusammengenom-

men Methylen oder Aethylen, die durch Hydroxy substituiert sein können, bedeuten. Von diesen sind diejenigen bevorzugt, in denen $R^8$ und $R^9$ zusammengenommen $-(CH_2)_3-$ und $R^4$ und $R^6$ zusammengenommen Methylen oder Hydroxymethylen darstellen.

In einer weiteren Ausführungsform betrifft die Erfindung **die Verwendung der** Verbindungen der Formel I, in denen $R^4$ bis $R^9$ Wasserstoff oder $C_{1-5}$-Alkyl darstellen. Von diesen sind diejenigen bevorzugt, in denen $R^4$ und $R^6$ Wasserstoff, $R^5$ und $R^7$ Wasserstoff oder $C_{1-5}$-Alkyl, insbesondere Methyl, und $R^8$ und $R^9$ $C_{1-5}$-Alkyl, insbesondere Methyl darstellen. Weiterhin sind Verbindungen der Formel I bevorzugt, in denen $R^2$ $C_{2-8}$-Alkyl oder $-OCH_2R^3$ und $R^3$ $C_{1-6}$-Alkyl darstellen. Von den Verbindungen der Formel I, in denen $R^1$ einen Rest (a) darstellt, sind diejenigen bevorzugt, in denen die punktierte Bindung im Rest (a) abwesend ist.

Von besonderem Interesse **ist**-schliesslich die **die Verwendung der** Verbindungen der Formel I, in denen $R^2$ n-$C_{2-8}$-Alkanoyl darstellt oder in denen $R^2$ einen Alkenyl- oder Alkinylrest darstellt oder enthält.

Die Verbindungen der Formel I und ihre Salze können dadurch hergestellt werden, dass man

a) eine Verbindung der allgemeinen Formel

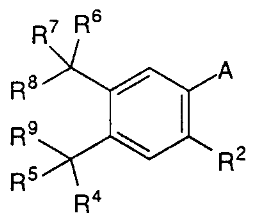

II

mit einer Verbindung der allgemeinen Formel

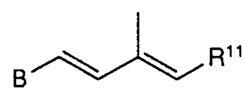

III

oder

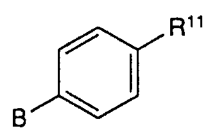

IV

umsetzt,

wobei eines der Symbole A und B $-CH_2P(O)(OAlk)_2$ oder $-CH_2P+(Ph)_3Y-$ und das andere Symbol A oder B Formyl ist; Ph Phenyl oder substituiertes Phenyl; Alk $C_{1-6}$-Alkyl; und Y- ein Anion und $R^{11}$ $C_{1-6}$-Alkoxy-carbonyl ist; und die übrigen Symbole die oben angegebene Bedeutung haben; oder

b) eine Verbindung der allgemeinen Formel

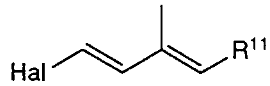

V

mit einer Verbindung der allgemeinen Formel

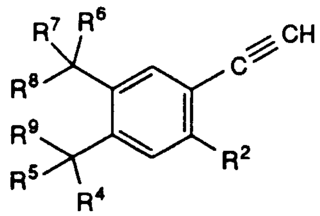

VI

umsetzt,
wobei Hal Brom oder Jod und $R^{11}$ $C_{1-6}$-Alkoxy-carbonyl ist; und die übrigen Symbole die oben angegebene Bedeutung haben; oder

c) eine Verbindung der allgemeinen Formel

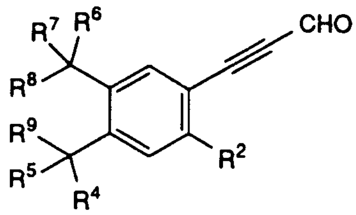

VII

mit einer Verbindung der allgemeinen Formel

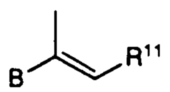

VIII

worin B $-CH_2P(O)(OAlk)_2$ oder $-CH_2P^+(Ph)_3Y^-$ ist und $R^{11}$ $C_{1-6}$-Alkoxycarbonyl ist,

umsetzt, oder

d) eine Verbindung der allgemeinen Formel

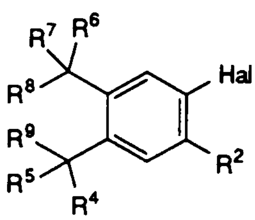

IX

mit einer Verbindung der allgemeinen Formel

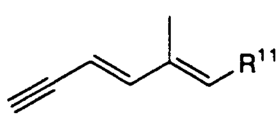

X

umsetzt, wobei Hal Brom oder Jod ist und die übrigen Symbole die oben angegebene Bedeutung haben, oder

e) in einer Verbindung der allgemeinen Formel

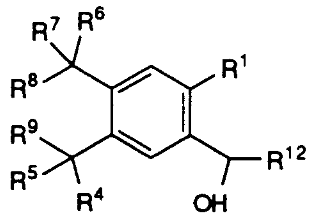

XI

worin $R^{12}$ $C_{1-7}$-Alkyl ist,

die Hydroxygruppe zur Oxogruppe oxidiert,

und gewünschtenfalls in der erhaltenen Verbindung der Formel I die Alkoxycarbonylgruppe in eine Carboxylgruppe oder ein pharmazeutisch anwendbares Salz davon, oder in eine mono- oder di-$C_{1-6}$(Alkyl)carbamoylgruppe umwandelt.

Die vorstehenden Umsetzungen können nach an sich bekannten Methoden vorgenommen werden.

Die Umsetzung der Verbindungen II und III gemäss Verfahrensvariante a) sowie VII und VIII gemäss Verfahrensvariante c) kann nach den bekannten Methoden der Wittig- bzw. Horner-Reaktion durchgeführt werden.

Bei der Wittig-Reaktion, d.h. bei Verwendung von Verbindungen der Formeln II und III mit A oder B = -$CH_2P^+(Ph)_3Y^-$ werden die Komponenten in Gegenwart eines säurebindenden Mittels, z.B. in Gegenwart einer starken Base, wie z.B. Butyllithium, Natriumhydrid oder dem Natriumsalz von Dimethylsulfoxid, oder K-tert.Butylat, vornehmlich aber in Gegenwart eines gegebenenfalls durch niederes Alkyl substituierten Aethylenoxyds wie 1,2-Butylenoxyd, gegebenenfalls in einem Lösungsmittel, z.B. in einem Aether, wie Diäthyläther oder Tetrahydrofuran, oder in einem aromatischen Kohlenwasserstoff, wie Benzol in einem zwischen etwa -20°C und dem Siedepunkt des Reaktionsgemisches liegenden Temperaturbereich miteinander umgesetzt.

Von den anorganischen Säureanionen $Y^-$ ist das Chlor- und Brom-ion oder das Hydrosulfat-ion, von den organischen Säureanionen ist das Tosyloxy-ion bevorzugt. Der Rest Ph ist vorzugsweise Phenyl.

Bei der Horner-Reaktion, d.h. bei Verwendung von Verbindungen der Formeln II oder III mit A oder B = -$CH_2P(O)(OAlk)_2$, werden die Komponenten mit Hilfe einer Base und vorzugsweise in Gegenwart eines inerten organischen Lösungsmittels, z.B. mit Hilfe von Natriumhydrid in Benzol, Toluol, Dimethylformamid, Tetrahydrofuran, Dioxan oder 1,2-Dimethoxyäthern, oder auch mit Hilfe eines Natriumalkoholates in einem Alkanol, z.B. Natriummethylat in Methanol, in einem zwischen etwa -20°C und dem Siedepunkt des Reaktionsgemisches liegenden Temperaturbereich kondensiert.

Die Kupplungsreaktionen b) und d) können, direkt von den Acetylenen VI bzw. X ausgehend, durch Phosphinkomplexe des Palladiums und Nickels und Cu(I)-Salze katalysiert werden. In allen Fällen ist die Gegenwart einer Base zweckmässig, beispielsweise einer organischen Stickstoffbase, wie Triäthylamin, Piperidin oder Pyridin, oder eines Alkalimetallalkoholats, wie Natriummethanolat oder Natriumphenolat. Gegebenenfalls wird in einem Lösungsmittel gearbeitet, vorzugsweise in Benzol, Dimethylformamid oder Tetrahydrofuran. Die Umsetzung verläuft zweckmässigerweise bei einer Temperatur von 20 bis 150°C.

Die Oxidation e) kann mit Oxidationsmitteln wie $MnO_2$ in einem inerten organischen Lösungsmittel, z.B. einem chlorierten Kohlenwasserstoff wie Methylenchlorid, vorzugsweise bei Raumtemperatur, durchgeführt werden.

Nach der Verfahrensvariante a) können Verbindungen der Formel I erhalten werden, in denen $R^1$ einen Rest der Formel (a) darstellt, in denen die punktierte Bindung abwesend ist; oder einen Rest der Formel (b) darstellt; und $R^2$ einen wie oben definierten Rest mit Ausnahme eines Alkanoylrests bedeutet.

Nach den Verfahrensvarianten b), c) und d) können Verbindungen der Formel I erhalten werden, in denen $R^1$ ein Rest (a) darstellt, in dem die punktiert dargestellte Bindung anwesend ist.

Nach der Verfahrensvariante e) werden Verbindungen erhalten, in denen $R^2$ einen $C_{2-8}$-Alkanoylrest darstellt.

In einer erhaltenen Verbindung der Formel I kann die Carbonsäureestergruppe zur Carboxylgruppe verseift und diese dann weiterhin in ein Salz oder ein Amid umgewandelt werden.

Ein Carbonsäureester der Formel I kann, wie nachstehend beschrieben, unmittelbar amidiert werden, oder in an sich bekannter Weise, z.B. durch Behandlung mit Alkalien, insbesondere durch Behandeln mit wässriger alkoholischer Natron- oder Kalilauge, in einem zwischen Raumtemperatur und dem Siedepunkt des Reaktionsgemisches liegenden Temperaturbereich zur Carbonsäure hydrolysiert werden, die wiederum über ein Säurehalogenid amidiert werden kann.

Eine Carbonsäure der Formel I kann in an sich bekannter Weise, z.B. durch Behandeln mit Thionylchlorid, oder Phosphortrichlorid in Toluol oder Oxalylchlorid in DMF/Benzol in das Säurechlorid übergeführt werden, das durch Umsetzen mit Alkoholen in Ester, mit Aminen in das entsprechende Amid umgewandelt werden kann.

Ein Carbonsäureester der Formel I kann z.B. durch Behandeln mit Lithiumamid direkt in das entsprechende Amid umgewandelt werden. Das Lithiumamid wird vorteilhaft bei Raumtemperatur mit dem betreffenden Ester zur Reaktion gebracht.

Alle diese Abwandlungen können nach an sich bekannten Methoden vorgenommen werden.

Beispiele von Salzen, in die die Carbonsäuren der Formel I übergeführt werden können, sind Alkalimetallsalze, wie Na- und K-Salze, Erdalkalimetallsalze wie Ca- und Mg-Salze, und Ammoniumsalze, z.B. Salze mit Alkylaminen und Hydroxyalkylaminen oder mit anderen organischen Basen, wie Dimethylamin, Diäthanolamin und Piperidin.

Die Verbindungen der Formel I können als E/Z-Isomerengemische anfallen, die nach an sich bekannten Methoden getrennt werden können. Bevorzugt sind die E-(all-E)-Isomeren.

Die Verbindungen der Formel II-X können, soweit sie nicht bekannt oder ihre Herstellung nachstehend beschrieben ist, in Analogie zu bekannten oder den nachstehend beschriebenen Verfahren hergestellt werden.

Die Verbindungen wirken als selektive Liganden des Retinsäure-$\gamma$-Rezeptors (RAR-$\gamma$). Sie **werden erfindungsgemäss** zur Therapie und Prophylaxe von licht- und altersbedingten Schäden der Haut sowie zur Förderung der Wundheilung beispielsweise von Schnittwunden, wie Opera tionswunden, Verbrennungswunden und anderen, von cutanen Traumata herrührenden Wunden verwendet. Die Eignung der Verbindungen für diesen Zweck kann in den in Science 237: 1333-1336 (1987) und J. Pathol. 129: 601-613 (1987) beschriebenen Modellen gezeigt werden. Weiterhin können die Verbindungen **erfindungsgemäss** zur Therapie und Prophylaxe von dermatologischen Erkrankungen, die mit Epithelläsionen einhergehen, z.B. Akne und Psoriasis, sowie malignen und prämalignen Epithelläsionen, Tumoren und präcancerösen Veränderungen der Schleimhäute in Mund, Zunge, Kehlkopf, Oesophagus, Blase, Cervix und Colon verwendet werden.

Die Verbindungen der Formel I und deren Salze können dementsprechend in Form pharmazeutischer Präparate Anwendung finden.

Die zur systemischen Anwendung dienenden Präparate können z.B. dadurch hergestellt werden, dass man eine Verbindung der Formel I oder ein Salz davon als wirksamen Bestandteil nichttoxischen, inerten, an sich in solchen Präparaten üblichen festen oder flüssigen Trägern zufügt.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektionslösungen geeignet.

Für die enterale und parenterale Verabreichung können die Verbindungen der Formel I in Mengen von etwa 1-100 mg, vorzugsweise 5-30 mg/Tag, an Erwachsene verabreicht werden.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise 0,3-2%ige, Salben oder Crèmen geeignet.

Den Präparaten kann gegebenenfalls ein Antioxydationsmittel, z.B. Tocopherol, N-Methyl-$\gamma$-tocopheramin sowie butyliertes Hydroxyanisol oder butyliertes Hydroxytoluol beigemischt werden.

Die Wirksamkeit der erfindungsgemässen Verbindungen bei der Behandlung lichtgeschädigter Haut zeigt sich in den nachstehend beschriebenen Versuchsanordnungen:

Behebung UV-B-verursachter Hautschäden bei der haarlosen Maus

Haarlose Mäuse (HRS/J Stamm, Jackson Labs, zu Beginn des Experiments 5-7 Wochen alt) wurden dreimal wöchentlich mit einer Anordnung von 8 Westinghouse Bestrahlungslampen (FS40), die etwa 20 cm oberhalb der Tiere plaziert waren, bestrahlt. Die Strahlungsdosis wurde durch ein handelsübliches phototherapeutisches Kontrollgerät kontrolliert. Die UV-B Dosis wurde so gewählt, dass die Dosierung selten 0,06J/$cm^2$ überschritt, ein minimales Erythem, jedoch keine Verbrennungen oder Narben verursachte. Nach einer Gesamtdosis von etwa 3,5J/$cm^2$ ergab sich eine durch histologischen Befund gesicherte signifikante Elastosis, die durch Messungen des Elastins mittels Radioimmunoassay für Desmosin in der gesamten Haut bestätigt wurde. Der Desmosingehalt erhöhte sich um das zwei- bis dreifache nach 3,5J/$cm^2$ UV-B Bestrahlung. Um die Hautschäden zu beheben, wurde die UV-Bestrahlung unterbrochen und Gruppen der Tiere wurden separat dreimal wöchentlich mit verschiedenen Dosen von Verbindungen der Formel I, in Aceton gelöst, behandelt. Die Lösungen wurden jede Woche frisch so hergestellt, dass die zu verabreichende Dosis in 100 $\mu$l Aceton enthalten war, und topisch auf ein Gebiet von etwa 10 $cm^2$ auf den Rücken der Tiere aufgebracht. Eine

Kontrollgruppe wurde nur mit Aceton behandelt.

Nach 10 Behandlungswochen wurden die Tiere getötet, Hautpräparate angefertigt und durch Luna-Färbung des Elastins das Ausmass der Wiederherstellung quantitativ gemessen. Bei diesem Versuchsmodell wurde die Wiederherstellung der Haut definiert als das Aussehen einer normalen Dermis, die sich von der Epidermis bis auf die Schicht von komprimiertem Elastin erstreckt. Das Ausmass der Wiederherstellung war durch die Breite dieser Zone gegeben. Das Gebiet der Zone auf einer Standardlänge des histologischen Schnitts wurde gemessen und die Resultate als Gesamtfläche in $mm^2$ pro 20 mikroskopische Felder ausgedrückt. Die Resultate sind in Tabelle I zusammengestellt.

Tabelle I

| | | **Reparaturzone** [$mm^2 \cdot 10^{-3}$] |
|---|---|---|
| Kontrollwert | | 1,2 ± 0,2 |
| Verbindung A, | 0,05 µg | 9,8 ± 2,8 |
| | 2 µg | 27,2 ± 2,6 |
| | 10 µg | 35,9 ± 7,5 |
| Kontrollwert | | 1,67 ± 0,78 |
| Verbindung B, | 0,5 µg | 15,6 ± 2,74 |
| | 2 µg | 20,5 ± 7,17 |
| Kontrollwert | | 1,61 ± 0,68 |
| Verbindung C, | 2 µg | 32,8 ± 6,72 |
| | 10 µg | 59,5 ± 6,71 |
| Kontrollwert | | 3,3 ± 1,1 |
| Verbindung D, | 0,5 µg | 15,5 ± 4,9 |
| | 2 µg | 19,1 ± 4,9 |
| | 10 µg | 46,5 ± 6,9 |
| Verbindung E, | 0,5 µg | 7,48 ± 1,7 |
| | 2 µg | 4,70 ± 2,0 |
| | 10 µg | 22,2 ± 8,3 |

Verbindung A: p-[(E)-2-(3-Hexyl-5,6,7,8-tetrahydro-5,5,8,8-tetramethyl-2-naphthyl)vinyl]benzoesäure
Verbindung B: 4-[(E)-2-(3-Butyl-5,5,8,8-tetramethyl-5,6,7,8-tetrahydronaphth-2-yl)vinyl]benzoesäure
Verbindung C: (all E)-7-(3-Hexyl-5,5,8,8-tetramethyl-5,6,7,8-tetrahydronaphthalin-2-yl)-3-methyl-hepta-2,4,6-triensäure
Verbindung D: p-[(E)-2-(3-Ethyl-5,6,7,8-tetrahydro-5,5,8,8-tetramethyl-2-naphthyl)vinyl]benzoesäure
Verbindung E: p-[(E)-2-(3-Octyl-5,6,7,8-tetrahydro-5,5,8,8-tetramethyl-2-naphthyl)vinyl]benzoesäure

Die **Herstellung der Verbindungen der Formel I** wird durch die nachstehenden Beispiele erläutert.

Beispiel 1

83 ml Hexylbenzol wurden unter Eiskühlung mit 1,8 g Aluminiumchlorid versetzt. Nach kurzem Rühren tropfte man eine Lösung von 40 g 2,5-Dichlor-2,5-dimethyl-hexan in 300 ml Schwefelkohlenstoff hinzu und liess weitere 2 Stunden bei 0°C rühren. Das Reaktionsgemisch wurde auf Eis/6N Salzsäure gegossen, mit Essigester extrahiert, über Natriumsulfat getrocknet und eingedampft. Das Rohprodukt wurde am Hochvakuum destilliert. Man erhielt 25,5 g 1,1,4,4-Tetramethyl-6-hexyl-1,2,3,4-tetrahydronaphthalin als farbloses Oel, Siedepunkt 141-152°C/0,8 mm.

10 g 1,1,4,4-Tetramethyl-6-hexyl-1,2,3,4-tetrahydro-naphthalin wurden in 100 ml Tetrachlorkohlenstoff gelöst und nach Zugabe einer Spatelspitze Eisenpulver bei 0°C tropfenweise mit 6,4 g Brom versetzt. Nach 3-stündigem Rühren bei 0°C wurde das Reaktionsgemisch auf Eiswasser gegossen, mit Aether extrahiert und nach dem Trocknen über Natriumsulfat eingedampft. Das Rohprodukt wurde durch Filtration über eine Kieselgelsäule (Eluierungsmittel Hexan/Essigester = 9:1) gereinigt und ergab 13 g 1,1,4,4-Tetramethyl-6-hexyl-7-brom-1,2,3,4-tetrahydro-naphthalin als

schwach gelbes Oel.

10 g dieses Bromides wurden in 100 ml abs. Tetrahydrofuran gelöst und bei -78°C tropfenweise mit 40 ml tert.Butyllithium, 1,4 molar in Pentan, versetzt. Nach 1-stündigem Rühren bei -78°C wurden 60 ml abs. Dimethylformamid hineingetropft. Es wurde noch 1 Stunde bei Raumtemperatur gerührt, dann auf Eis gegossen, mit 2N Salzsäure angesäuert und mit Aether extrahiert. Die organischen Phasen wurden getrocknet, eingedampft und das Rohprodukt über eine Kieselgelsäule (Eluierungsmittel Hexan/Essigester = 19:1) filtriert. Man erhielt 6,8 g 2-Formyl-3-hexyl-5,5,8,8-tetramethyl-5,6,7,8-tetrahydro-naphthalin als gelbliches Oel.

3,2 g Natriumhydrid, 50% in Mineralöl, wurden zweimal mit Pentan gewaschen und in 50 ml abs. Dimethylsulfoxid suspendiert. Nach dem Zutropfen einer Lösung von 20 g Diäthyl (4-carbäthoxybenzyl)phosphonat in 50 ml Dimethylsulfoxid bei ca. 15°C wurde das Reaktionsgemisch 2 weitere Stunden bei Raumtemperatur gerührt. Danach tropfte man eine Lösung von 9 g 2-Formyl-3-hexyl-5,5,8,8-tetramethyl-5,6,7,8-tetrahydro-naphthalin in 50 ml abs. Dimethylsulfoxid hinzu und erwärmte 3 Stunden auf 40°C. Das Reaktionsgemisch wurde anschliessend auf Eiswasser gegossen, mit 2N Salzsäure angesäuert und mit Aether extrahiert. Nach dem Trocknen der organischen Phase wurde eingedampft und das Rohprodukt über eine Kieselgelsäule (Eluierungsmittel Hexan/Essigester = 19:1) filtriert. Man erhielt 5,0 g Aethyl p-[(E)-2-(3-hexyl-5,6,7,8-tetrahydro-5,5,8,8-tetramethyl-2-naphthyl)vinyl]benzoat als gelbliches Oel.

8 g dieses Esters wurden in 50 ml Aethanol gelöst und mit einer Lösung von 10 g Kaliumhydroxid in 10 ml Wasser und 20 ml Aethanol versetzt. Nach Zugabe von 50 ml Tetrahydrofuran wurde während 4 Stunden auf 40°C erwärmt. Die klare, gelbe Reaktionslösung wurde auf ein Gemisch aus Eis und 6N Salzsäure gegossen, mit Essigester extrahiert, die organische Phase mit Wasser gewaschen, getrocknet und eingedampft. Nach dem Umkristallisieren des kristallinen Rückstandes aus Hexan/Essigester erhielt man 6,2 g p-[(E)-2-(3-Hexyl-5,6,7,8-tetrahydro-5,5,8,8-tetramethyl-2-naphthyl)vinyl]benzoesäure in weissen Kristallen, Schmelzpunkt 134-136°C.

Beispiel 2

In Analogie zu Beispiel 1 wurde ausgehend von Pentylbenzol die (E)-4-[2-(3-Pentyl-5,5,8,8-tetramethyl-5,6,7,8-tetrahydro-naphthalin-2- yl)vinyl]benzoesäure hergestellt. Weisse Kristalle, Schmelzpunkt 173-174°C (aus Essigester).

Beispiel 3

In Analogie zu Beispiel 1 wurde ausgehend von Butylbenzol die (E)-4-[2-(3-Butyl-5,5,8,8-tetramethyl-5,6,7,8-tetrahydro-naphthalin-2- yl)vinyl]benzoesäure hergestellt. Weisse Kristalle, Schmelzpunkt 192-194°C (aus Essigester/Hexan).

Beispiel 4

4,1 g Natriumhydrid, 50% in Mineralöl, wurden mit Pentan gewaschen und in 70 ml abs. Dimethylsulfoxid suspendiert. Nach dem Zutropfen einer Lösung von 24,5 g (all-E)-6-(Diäthoxyphosphinyl)-3-methyl-2,4-hexadiencarbonsäureäthylester in 70 ml abs. Dimethylsulfoxid bei 15°C wurde das Reaktionsgemisch 1/2 Stunde bei Raumtemperatur gerührt. Danach tropfte man bei 20°C eine Lösung von 11,3 g 2-Formyl-3-hexyl-5,5,8,8-tetramethyl-5,6,7,8-tetrahydronaphthalin in 70 ml Dimethylsulfoxid und 35 ml Tetrahydrofuran hinzu und rührte weitere 2 Stunden bei Raumtemperatur. Das rotbraune Reaktionsgemisch wurde anschliessend auf Eiswasser gegossen, mit 80 ml 1N Salzsäure angesäuert und mit Essigester extrahiert. Das orange Rohprodukt wurde durch Flashchromatographie (Kieselgel, Eluierungsmittel Hexan/2% Methyl-tert.butyläther) gereinigt. Man erhielt 8,5 g (all-E)-7-(3-Hexyl-5,5,8,8-tetramethyl-5,6,7,8-tetrahydro-naphthalin-2-yl)-3-methyl-hepta-2,4,6-triencarbonsäureäthylester als schwach gelbliches Oel. Als Nebenprodukt (unpolarere Verbindung) wurden 4,1 g (2Z,4E,6E)-7-(3-Hexyl-5,5,8,8-tetramethyl-5,6,7,8-tetrahydronaphthalin-2-yl)-3- methylhepta-2,4,6-triencarbonsäuremethylester als schwach gelbliches Oel isoliert.

8,5 g all-trans-Ester wurden in 160 ml Aethanol und 30 ml Tetrahydrofuran gelöst und mit einer Lösung von 12,8 g Kaliumhydroxid in 60 ml Wasser versetzt. Nach 3-stündigem Rühren bei 45°C wurde die Hauptmenge Aethanol abdestilliert, der Rückstand auf Eis gegossen und mit 1N Salzsäure angesäuert. Nach Extraktion mit Essigester, Trocknen und Eindampfen wurde das Rohprodukt aus Essigester/Hexan umkristallisiert. Man erhielt 5,6 g (all-E)-7-(3-Hexyl-5,5,8,8-tetramethyl-5,6,7,8-tetrahydronaphthalin-2-yl)-3-methyl-hepta-2,4,6-triencarbonsäure in schwach gelben Kristallen, Schmelzpunkt 173-174°C.

Beispiel 5

In Analogie zu Beispiel 4 wurde ausgehend von 2-Formyl-3-pentyl-5,5,8,8-tetramethyl-5,6,7,8-tetrahydro-naphthalin die (2E,4E,6E)-7-(3-Pentyl-5,5,8,8-tetramethyl-5,6,7,8-tetrahydro-naphthalin-2-yl)-3-methyl-hepta-2,4,6-triencarbonsäure hergestellt. Blassgelbe Kristalle. Schmelzpunkt 164-166°C (aus Essigester/Hexan).

Beispiel 6

Durch Verseifung des entsprechenden Aethylesters aus Beispiel 4 wurde die (2Z,4E,6E)-7-(3-Pentyl-5,5,8,8-tetramethyl-5,6,7,8-tetrahydronaphthalin-2-yl)-3-methyl-hepta-2,4,6-triencarbonsäure hergestellt. Blassgelbe Kristalle, Schmelzpunkt 170-174°C (aus Essigester/Hexan).

Beispiel 7

1,4 g Natriumhydrid, 50% in Mineralöl, wurden in 50 ml abs. Dimethylsulfoxid suspendiert und unter Eiskühlung tropfenweise mit 8,5 g 2-Brom-3-hydroxy-5,5,8,8-tetramethyl-5,6,7,8-tetrahydro-naphthalin gelöst in 60 ml abs. Dimethylformamid versetzt. Das Reaktionsgemisch wurde während 1/2 Stunde bei 40°C gerührt und anschliessend unter Eiskühlung eine Lösung von 4,4 g Propylbromid in 20 ml Dimethylformamid hinzugetropft. Nach 20-stündigem Rühren bei Raumtemperatur wurde auf Eiswasser gegossen, mit Essigester extrahiert und das nach Trocknen und Eindampfen des Lösungsmittels erhaltene Rohprodukt über eine Kieselgelsäule (Eluierungsmittel Hexan/Essigester = 9:1) filtriert. Man erhielt 9,5 g 2-Brom-3-propoxy-5,5,8,8-tetramethyl-5,6,7,8-tetrahydro-naphthalin als bräunliches Oel.
Dieses Oel wurde in 100 ml abs. Tetrahydrofuran gelöst und bei -78°C tropfenweise mit 40 ml tert.Butyllithium, 1,4 molar in Pentan, versetzt. Nach 1/2-stündigem Rühren bei -78°C wurden 75 ml abs. Dimethylformamid zugetropft und bei Raumtemperatur 1 Stunde gerührt. Die hellbeige Suspension wurde auf Eiswasser gegossen und mit Essigester extrahiert. Nach Trocknen und Eindampfen der organischen Phase wurde das Rohprodukt über eine Kieselgelsäule (Eluierungsmittel Hexan/5% Aether) filtriert. Man erhielt 6,6 g 2-Formyl-3-propoxy-5,5,8,8-tetramethyl-5,6,7,8-tetrahydronaphthalin als hellgelbes Oel, das beim Kühlen erstarrte, Schmelzpunkt 51-52°C.

1,3 g Natriumhydrid, 50% in Mineralöl, wurden in 20 ml abs. Dimethylsulfoxid (DMSO) suspendiert und bei 15°C tropfenweise mit einer Lösung von 8,6 g Diäthyl (4-carbäthoxybenzyl)phosphonat in 25 ml abs. DMSO versetzt. Man rührte 1 Stunde bei Raumtemperatur und tropfte dann eine Lösung von 3,3 g des obigen Aldehydes in 25 ml abs. DMSO und 10 ml Tetrahydrofuran hinzu. Nach 2-stündigem Rühren bei 40°C goss man auf Eiswasser, säuerte mit 2N Salzsäure an und extrahierte mit Essigester. Nach dem Trocknen der organischen Phase mit Natriumsulfat und Eindampfen des Lösungsmittels wurde das Rohprodukt über eine Kieselgelsäule (Eluierungsmittel Hexan/5% Methyl-tert.butyläther) filtriert. Man erhielt nach Umkristallisation aus Hexan/Essigester 4,4 g (E)-4-[2-(5,5,8,8-Tetramethyl-3-propoxy-5,6,7,8-tetrahydro-naphthalin-2- yl)vinyl]benzoesäureäthylester in weissen Kristallen, Schmelzpunkt 97-100°C.

Verseifung dieses Esters mit einer Lösung von 6,8 g Kaliumhydroxid in 34 ml Wasser, 85 ml Aethanol und 20 ml Tetrahydrofuran bei 45°C während 4 Stunden ergab nach Ansäuern mit 2N Salzsäure, Extraktion mit Essigester und Umkristallisation aus Essigester 3,5 g (E)-4-[2-(5,5,8,8-Tetramethyl-3-propoxy-5,6,7,8-tetrahydro-naphthalin-2-yl)vinyl]benzoesäure in weissen Kristallen, Schmelzpunkt 192°C.

Beispiel 8

In Analogie zu Beispiel 7 wurde ausgehend von 2-Brom-3-hydroxy-5,5,8,8-tetramethyl-5,6,7,8-tetrahydro-naphthalin und Pentylbromid die (E)-4-[2-(5,5,8,8-Tetramethyl-3-pentoxy-5,6,7,8-tetrahydro-naphthalin-2-yl)vinyl]benzoesäure hergestellt. Weisse Kristalle, Schmelzpunkt 148-149°C (aus Essigester).

Beispiel 9

In Analogie zu Beispiel 4 wurden aus 8,4 g (all-E)-6-Dimethoxyphosphinyl-3-methyl-2,4-hexadiencarbonsäureäthylester und 3,3 g 2-Formyl-3-propoxy-5,5,8,8-tetramethyl-5,6,7,8-tetrahydro-naphthalin 2,7 g (2E,4E,6E)-3-Methyl-7-(5,5,8,8-tetramethyl-3-propoxy-5,6,7,8-tetrahydro-naphthalin-2-yl)-hepta-2,4,6-triencarbonsäureäthylester, Schmelzpunkt 92-93°C (aus Hexan) und 1,5 g des entsprechenden 2Z-Isomeren als gelbes Oel isoliert.

Verseifung des 2E-Isomeren ergab nach Umkristallisation aus Essigester (2E,4E,6E)-3-Methyl-7-(5,5,8,8-tetramethyl-3-propoxy-5,6,7,8-tetrahydronaphthalin-2-yl)-hepta-2,4,6-triencarbonsäure in gelben Kristallen, Schmelzpunkt 186°C.

Verseifung des 2Z-Isomeren ergab nach Umkristallisation aus Essigester (2Z,4E,6E)-3-Methyl-7-(5,5,8,8-tetramethyl-3-propoxy-5,6,7,8-tetrahydronaphthalin-2-yl)-hepta-2,4,6-triencarbonsäure als gelbe Kristalle vom Schmelzpunkt 210-211°C.

Beispiel 10

In Analogie zu Beispiel 9 wurde ausgehend von 2-Formyl-3-pentoxy-5,5,8,8-tetramethyl-5,6,7,8-tetrahydro-naphthalin die (2E,4E,6E)-3-Methyl-7-(5,5,8,8-tetramethyl-3-pentoxy-5,6,7,8-tetrahydro-naphthalin-2-yl)-hepta-2,4,6-triencarbonsäure, gelbe Kristalle, Schmelzpunkt 162-163°C (aus Essigester) und, als Nebenprodukt, die (2Z,4E,6E)-3-Methyl-7-(5,5,8,8-tetramethyl-3-pentoxy-5,6,7,8-tetrahydro-naphthalin-2-yl)-hepta-2,4,6- triencarbonsäure, gelbe Kristalle, Schmelzpunkt 168-169°C (aus Essigester) erhalten.

Beispiel 11

Zu 3,76 g 7,7-Dimethyl-2-octyl-6,7,8,9-tetrahydro-5H-benzocyclohepten in 30 ml Methylenchlorid wurde unter Argon eine Spatelspitze Fe-Pulver und dann bei 0°C 680 µl Brom, gelöst in 20 ml Methylenchlorid gegeben. Nach 5 Stunden wurde das Reaktionsgemisch auf Eis gegossen, mit Aethylacetat extrahiert, mit Natriumbisulfitlösung gewaschen, getrocknet und eingedampft. Das Rohprodukt (4,43 g) enthielt 91% 2-Brom-3-octyl-7,7-dimethyl-6,7,8,9-tetrahydro-5H-benzocyclohepten neben 7% Regioisomeren und wurde roh wie folgt weiter verarbeitet:

Das Rohprodukt wurde unter Argon in 25 ml abs. Tetrahydrofuran gelöst und bei -78°C langsam mit 7,51 ml 1,55M n-Butyllithium versetzt. Nach 15 Minuten bei dieser Temperatur wurden 2,37 ml abs. Dimethylformamid zugesetzt und das Reaktionsgemisch auf Raumtemperatur erwärmt. Danach wurde auf Eis gegossen, mit Diäthyläther extrahiert, mit Natriumchloridlösung gewaschen, getrocknet und eingedampft. Flashchromatographie an $SiO_2$ (Hexan/Aethylacetat 97:3) ergab 2,38 g Aldehyd als farbloses Oel, der wie folgt in einer Wittig-Reaktion eingesetzt wurde:

Zu 383 mg Natriumhydrid (ca. 50% in Oel) in 18 ml abs. Dimethylformamid wurden unter Argon bei 0°C 3,56 g Diäthyl (4-carbäthoxybenzyl)phosphonat gegeben. Das Reaktionsgemisch wurde dann ca. 2 Stunden bei Raumtemperatur gerührt, bis die Wasserstoffentwicklung beendetet war. Danach wurde auf -10°C gekühlt und der oben hergestellte Aldehyd, in 4 ml abs. Dimethylformamid gelöst, langsam zugetropft. Danach entfernte man das Kühlbad und liess 3 Stunden bei Raumtemperatur nachreagieren. Das Reaktionsgemisch wurde auf Eis gegossen, mit Diäthyläther extrahiert, mit Wasser gewaschen, getrocknet und eingedampft. Flashchromatographie an $SiO_2$ (Hexat/Aethylacetat 98:2) ergab 2,01 g (E)-4-[2-(7,7-Dimethyl-3-octyl-6,7,8,9-tetrahydro-5H-benzocyclohepten-2- yl)vinyl]benzoesäureäthylester als farbloses Oel, der folgendermassen hydrolysiert wurde:

Der Ester wurde in 20 ml Aethanol/Tetrahydrofuran (1:1) gelöst und mit 5,8 ml 3N NaOH versetzt. Danach wurde 3 Tage bei Raumtemperatur gerührt, auf Eis gegossen, mit 2H HCl angesäuert, mit Aethylacetat extrahiert, mit Wasser gewaschen, getrocknet und eingedampft. Umkristallisation aus Aethylacetat lieferte 1,56 g (E)-4-[2-(7,7-Dimethyl-3-octyl-6,7,8,9-tetrahydro-5H-benzocyclohepten-2-yl)vinyl]benzoesäure als blassgelbe Kristalle vom Schmelzpunkt 179,5-180,5°C.

Beispiel 12

In Analogie zu Beispiel 11 wurde aus 7,7,9-Trimethyl-2-octyl-6,7,8,9-tetrahydro-5H-benzocyclohepten die rac-(E)-4-[2-(5,7,7-Trimethyl-3-octyl-6,7,8,9-tetrahydro-5H-benzocyclohepten-2-yl)vinyl]benzoesäure als blassgelbe Kristalle vom Schmelzpunkt 182-183°C erhalten.

Das Ausgangsmaterial wurde aus 7,7-Dimethyl-3-octyl-6,7,8,9-tetrahydro-5H-benzocyclohepten-5-on durch Addition von Methylmagnesiumjodid in Diäthyläther und nachfolgende Deoxygenierung mit Trimethylsilylchlorid/Natriumjodid/Acetonitril/Hexan hergestellt.

Beispiel 13

In Analogie zu Beispiel 11 wurde aus 2-Butyl-9,9-dimethyl-6,7,8,9-tetrahydro-5H-benzocyclohepten die (E)-4-[2-(3-Butyl-5,5-dimethyl-6,7,8,9-tetrahydro-5H-benzocyclohepten-2-yl)vinyl]benzoesäure als weisse Kristalle vom Schmelzpunkt 184-186°C erhalten.

Das Ausgangsmaterial wurde wie folgt hergestellt:

Unter Argon wurde aus 304 mg Mg-Spänen und 2,50 g 2-Brom-9,9-dimethyl-6,7,8,9-tetrahydro-5H-benzocyclohepten in 25 ml abs. Tetrahydrofuran die entsprechende Grignard-Verbindung hergestellt. Nach Kühlen auf -15°C wurden nacheinander 206 mg CuI und 1,78 ml 1-Jodbutan zugegeben. Das Gemisch wurde 2 Stunden bei Raumtemperatur reagieren gelassen und dann auf Eis/Ammoniumchloridlösung gegossen. Die Lösung wurde mit Diäthyläther extrahiert, mit Wasser gewaschen, getrocknet und eingedampft. Nach Filtration über $SiO_2$ mit Hexan als Elutionsmittel wurden 2,27 g 2-Butyl-9,9-dimethyl-6,7,8,9-tetrahydro-5H-benzocyclohepten (verunreinigt mit 4% Regioisomeren und 6% 5,5-Dimethyl-6,7,8,9-tetrahydro-5H-benzocyclohepten) erhalten.

Analog wurden die folgenden Verbindungen erhalten:

(E)-4-[2-(3-Hexyl-7,7-dimethyl-indan-2-yl)vinyl]benzoesäure, weisse Kristalle vom Schmelzpunkt 149-150°C,
(E)-4-[2-(3-Hexyl-5,5-dimethyl-6,7,8,9-tetrahydro-5H-benzocyclohepten-2-yl)vinyl]benzoesäure, farblose Kristalle vom Schmelzpunkt 133-134°C und
(E)-4-[2-(3-Butyl-9,9-dimethyl-6,7,8,9-tetrahydro-5H-benzocyclohepten-2-yl)vinyl]benzoesäure, weisse Kristalle

vom Schmelzpunkt 81-83°C.

Beispiel 14

Unter Argon wurden in 12 ml abs. DMF 248 mg NaH (ca. 50% in Oel) vorgelegt. Man gab bei 0° 2,45 g Diäthyl (4-carbäthoxybenzyl)phosphonat zu und rührte anschliessend ca. 2 Stunden bei Raumtemperatur, bis die $H_2$-Entwicklung beendet war. Man kühlte auf -10° und tropfte 2,20 g (5RS,9SR,10RS)-10-t-Butyldimethylsilyloxy-2-formyl-3-heptyloxy-5,9-dimethyl-6,7,8,9-tetrahydro-5,9-methano-5H-benzocyclohepten, gelöst in 10 ml abs. DMF, langsam zu. Dann entfernte man das Kühlbad und liess 2 Stunden bei Raumtemperatur nachreagieren. Man goss auf Eis/$NH_4Cl$-Lösung, extrahierte mit Diäthyläther, wusch mit NaCl-Lösung, trocknete über $Na_2SO_4$ und engte ein. Flash-Chromatographie an $SiO_2$ (Hexan/Aethylacetat 98/2) ergab 2,32 g (E)-4-[2-[(5RS,9SR,10RS)-10-t-Butyldimethylsilyloxy-3-heptyloxy-5,9-dimethyl-6,7,8,9-tetrahydro-5,9-methano-5H- benzocyclohepten-2-yl]vinyl]benzoesäureäthylester als gelbliches Oel.

Dieses wurde in 12 ml abs. THF gelöst und mit 3,72 g Tetrabutylammoniumfluorid versetzt. Man rührte über Nacht bei 35° und goss dann auf Eis. Man extrahierte mit Diäthyläther, wusch mit Wasser, trocknete über $Na_2SO_4$ und rotierte ein. Flash-Chromatographie an $SiO_2$ (Hexan/Aethylacetat 85/15), gefolgt von Kristallisation aus wenig Hexan, lieferte 1,44 g (E)-4-[2-[(5RS,9SR,10RS)-3-Heptyloxy-10-hydroxy-5,9-dimethyl-6,7,8,9-tetrahydro-5,9-methano-5H-benzocyclohepten-2- yl]vinyl]benzoesäureäthylester als gelbliche Kristalle vom Schmelzpunkt 78-81°C.

1,255 g (E)-4-[2-[(5RS,9SR,10RS)-3-Heptyloxy-10-hydroxy-5,9-dimethyl-6,7,8,9-tetrahydro-5,9-methano-5H-benzocyclohepten-2- yl]vinyl]benzoesäureäthylester wurden in 30 ml Aethanol/THF = 1/1 gelöst und mit 2,56 ml 3N NaOH versetzt. Man rührte 5 Stunden bei Raumtemperatur, goss dann auf Eis/HCl konz., extrahierte mit Aethylacetat, wusch mit Wasser, trocknete über $Na_2SO_4$ und dampfte ein. Umkristallisation aus Hexan/Aethylacetat lieferte 873 mg (E)-4-[2-[(5RS,9SR,10RS)-3-Heptyloxy-10-hydroxy-5,9-dimethyl-6,7,8,9-tetrahydro-5,9-methano-5H-benzocyclohepten-2-yl]vinyl]benzoesäure als gelbe Kristalle vom Schmelzpunkt 89-90°C.

Das Ausgangsmaterial wurde wie folgt hergestellt:

4-Bromheptyloxybenzol und 2,6-Dimethylcyclohexanon wurden mit $NaNH_2$ zu (5RS,9SR,10RS)-2-Heptyloxy-10-hydroxy-5,9-dimethyl-6,7,8,9-tetrahydro-5,9-methano-5H-benzocyclohepten umgesetzt, das mit $Br_2$ und Eisen als Katalysator bromiert wurde.

4,49 g des so erhaltenen (5RS,9SR,10RS)-2-Brom-3-heptyloxy-10-hydroxy-5,9-dimethyl-6,7,8,9-tetrahydro-5,9-methano-5H- benzocycloheptens wurden unter Argon in 12 ml $CH_2Cl_2$ vorgelegt und mit 2,58 ml 2,6-Lutidin, gefolgt von 3,08 ml t-Butyldimethylsilyl-triflat, versetzt. Man rührte über Nacht bei 35°C und goss dann auf Eis. Man extrahierte mit Diäthyläther, wusch mit 1N HCl und Wasser, trocknete über $Na_2SO_4$ und rotierte ein. Filtriersäule ($SiO_2$/Hexan) ergab 5,39 g (5RS,9SR,10RS)-2-Brom-10-t-butyldimethylsilyloxy-3-heptyloxy-5,9-dimethyl-6,7,8,9-tetrahydro-5,9-methano-5H-benzocyclohepten als farbloses Oel.

4,85 g davon wurden unter Argon in 25 ml abs. THF vorgelegt und bei -78°C mit 7,0 ml 1,5M nBuLi (Hexan) versetzt. Man hielt das Reaktionsgemisch 1/4 Stunde bei dieser Temperatur, dann tropfte man 2,20 ml abs. DMF unverdünnt zu und entfernte anschliessend das Kühlbad. Nach 1/2 Stunde bei Raumtemperatur goss man auf Eis, extrahierte mit Diäthyläther, wusch mit Wasser, trocknete über $Na_2SO_4$ und dampfte ein. Flash-Chromatographie an $SiO_2$ (Hexan/Aethylacetat 8/2) ergab 4,01 g (5RS,9SR,10RS)-10-t-Butyldimethylsilyloxy-2-formyl-3-heptyloxy-5,9-dimethyl-6,7,8,9- tetrahydro-5,9-methano-5H-benzocyclohepten als farblose Kristalle vom Schmelzpunkt 66.67°C.

Beispiel 15

In Analogie zu Beispiel 14 wurden der (E)-4-[2-[(5RS,9SR,10RS)-3-Butoxy-10-hydroxy-5,9-dimethyl-6,7,8,9-tetrahydro-5,9-methano-5H- benzocyclohepten-2-yl]vinyl]benzoesäureäthylester als farblose Kristalle vom Schmelzpunkt 104-106°C, und daraus die (E)-4-[2-[(5RS,9SR,10RS)-3-Butoxy-10-hydroxy-5,9-dimethyl-6,7,8,9-tetrahydro-5,9-methano-5H- benzocyclohepten-2-yl]vinyl]benzoesäure als weisse Kristalle vom Schmelzpunkt 129-130°C erhalten.

Beispiel 16

4,6 g Aethyl p-[(E)-2-(3-hexyl-5,6,7,8-tetrahydro-5,5,8,8-tetramethyl-2-naphthyl)vinyl]benzoat wurden in 140 ml Tetrachlorkohlenstoff gelöst und nach Zugabe von 2 g N-Bromsuccinimid und einer Spatelspitze $\alpha,\alpha'$-Azoisobutyronitril während 1 Stunde am Rückfluss erhitzt. Nach dem Abkühlen wurde das ausgefallene Succinimid abfiltriert, die organische Phase eingeengt und der ölige Rückstand über Alox neutral filtriert. Man erhielt 5,6 g eines gelben, viskosen Oeles.

Dieses Oel (5,6 g) wurde in 125 ml Essigsäure gelöst und mit 2,5 g Silberacetat versetzt. Nach 7-stündigem Rühren bei Raumtemperatur wurde die Suspension über Dicalite filtriert, mehrfach mit tert.Butylmethyläther gewaschen, das Filtrat mit Eiswasser versetzt und mit tert.Butylmethyläther extrahiert. Nach dem Waschen der organischen Phase mit Wasser, Natriumbicarbonat- und Natriumchlorid-Lösung wurde über Natriumsulfat getrocknet und eingedampft. Man erhielt

5,5 g eines gelben Oeles, das durch Chromatographie (Kieselgel, Eluierungsmittel Hexan/5% Essigester) weiter gereinigt wurde und 3,8 g (E)-(RS)-4-[2-[3-(1-Acetoxy-hexyl)-5,5,8,8-tetramethyl-5,6,7,8-tetrahydro-naphthalin-2-yl]vinyl]benzoesäureäthylester als hellgelbes, viskoses Oel ergab. 1,4 g dieser Aethoxyverbindung wurden in 36 ml Tetrahydrofuran gelöst und nach Zugabe von 36 ml Methanol mit 280 mg fein pulverisiertem Kaliumcarbonat versetzt. Nach 8-stündigem Rühren bei Raumtemperatur wurde bei 30°C eingedampft und das Rohprodukt durch Chromatographie (Kieselgel, Eluierungsmittel Hexan/tert.-Butylmethyläther = 8:2) gereinigt. Man erhielt 1,1 g eines hellgelben Oeles. (Verbindung XI: $R^1$ = (b); $R^{10}$ = Aethoxycarbonyl; $R^{12}$ = n-Pentyl; $R^8$ + $R^9$ = Aethylen; $R^4$-$R^7$ = Methyl).

1,5 g dieses Oeles wurden in 40 ml Methylenchlorid gelöst und mit 3 g Mangandioxid versetzt. Nach 8-stündigem Rühren bei Raumtemperatur wurden erneut 3 g Mangandioxid zugegeben und weitere 22 Stunden gerührt. Nach Filtration über Dicalite wurde eingedampft und der ölige Rückstand über Kieselgel filtriert (Eluierungsmittel Hexan/tert.Butylmethyläther = 9:1). Man erhielt 1,4 g eines gelben Oeles, das in einem Gemisch von 50 ml Aethanol und 25 ml Tetrahydrofuran gelöst wurde und mit einer Lösung von 2,3 g Kaliumhydroxid in 20 ml Wasser und 20 ml Aethanol versetzt wurde. Nach 4-stündigem Rühren bei Raumtemperatur wurde mit 2N Salzsäure angesäuert und mehrfach mit Essigester extrahiert. Nach Waschen, Trocknen und Eindampfen erhielt man 1,4 g eines kristallinen Materials, das aus Hexan/Essigester umkristallisiert wurde und 1,1 g (E)-4-[2-(3-Hexanoyl-5,5,8,8-tetramethyl-5,6,7,8-tetrahydronaphthalin-2-yl)vinyl]benzoesäure in farblosen Kristallen ergab, Schmelzpunkt 118-119°C.

Beispiel 17

10 g 1,1,4,4-Tetramethyl-6-pentyl-7-brom-1,2,3,4-tetrahydro-naphthalin wurden in 47 ml Piperidin gelöst und unter Argon nacheinander mit 450 mg Tetrakis(triphenylphosphin)palladium, 88 mg Kupfer(I)jodid und 130 mg Triphenylphosphin versetzt. Im Laufe von 3,5 Stunden wurde bei einer Innentemperatur von 95°C eine Lösung von 8,6 ml Aethinyltrimethylsilan in 47 ml Piperidin hinzugetropft. Nach 4-stündigem Rühren bei 95°C wurden erneut 1,5 ml Aethinyl-trimethylsilan langsam zugetropft und das Reaktionsgemisch weitere 15 Stunden bei 95°C gerührt.Nach dem Abkühlen wurde auf Eis gegossen, mit 37% Salzsäure angesäuert und mit Hexan extrahiert. Nach dem Waschen, Trocknen und Eindampfen erhielt man ein rotes Oel, das nach Filtration über Kieselgel (Eluierungsmittel Hexan) 7,9 g eines hellgelben Oeles ergab.

8,1 g dieses Oeles wurden in 80 ml Tetrahydrofuran gelöst und mit 8 g Tetrabutylammoniumfluorid versetzt. Nach 1/2-stündigem Rühren bei Raumtemperatur wurde auf Eiswasser gegossen und mit Hexan extrahiert. Das nach Trocknen und Eindampfen erhaltene rotbraune Oel wurde über Kieselgel filtriert (Eluierungsmittel Hexan) und ergab 5,8 g eines hellbeigen Oeles.

Dieses Oel (5,8 g) wurde in 25 ml Tetrahydrofuran gelöst und unter Trockeneiskühlung tropfenweise mit 15,5 ml n-Butyllithium (1,6 molar in Hexan) versetzt. Nach 1-stündigem Rühren bei -78°C wurden 15,5 ml Dimethylformamid hinzugetropft und 1 Stunde bei Raumtemperatur gerührt. Das Reaktionsgemisch wurde anschliessend auf Eiswasser gegossen, mit 3N Salzsäure angesäuert und mit Essigester extrahiert. Das nach Trocknen und Eindampfen erhaltene orange Oel wurde über Kieselgel filtriert (Eluierungsmittel Hexan/Essigester = 8:2) und ergab 5,8 g eines gelben Oeles, das in der Kälte kristallisierte. (Verbindung II: A = Formyl, $R^2$ = n-Pentyl, $R^8$ + $R^9$ = Aethylen, $R^4$-$R^7$ = Methyl.)

2 g Natriumhydrid (50% in Mineralöl) wurden 2 mal mit Pentan gewaschen, in 38 ml Dimethylsulfoxid suspendiert und bei 15°C tropfenweise mit einer Lösung von 11,3 g 4-Diäthoxyphosphinyl-3-methyl-crotonsäureäthylester in 38 ml Dimethylsulfoxid versetzt. Nach 1-stündigem Rühren bei Raumtemperatur wurde eine Lösung von 5,8 g des substituierten Propargylaldehydes (wie oben beschrieben) in 40 ml Tetrahydrofuran hinzugetropft. Das Reaktionsgemisch wurde 2 Stunden bei Raumtemperatur gerührt, anschliessend auf Eiswasser gegossen, mit 2N Salzsäure angesäuert und mit Essigester extrahiert. Das nach Trocknen und Eindampfen erhaltene dunkelrote Oel wurde zunächst über Kieselgel filtriert (Eluierungsmittel Hexan/5% tert.Butylmethyläther) und anschliessend mit Mitteldruckchromatographie (Eluierungsmittel Hexan/2% tert.Butylmethyläther) aufgetrennt. Man erhielt 1,8 g (2E,4E)-3-Methyl-7-(5,5,8,8-tetramethyl-3-pentyl-5,6,7,8-tetrahydronaphthalin-2-yl)-hepta-2,4-dien-6-in- säureäthylester und 1,2 g der entsprechenden (2Z,4E)-Verbindung als gelbliche Oele.

1,8 g der (2E,4E)-Verbindung wurde in 36 ml Aethanol und 15 ml Tetrahydrofuran gelöst und mit einer Lösung von 2,8 g Kaliumhydroxid in 13 ml Wasser versetzt. Nach 2-stündigem Rühren bei 50°C wurde das Reaktionsgemisch zur Hälfte eingedampft, auf Eiswasser gegossen, mit 1N Salzsäure angesäuert und mit Essigester extrahiert. Das nach Trocknen und Eindampfen erhaltene kristalline Produkt wurde aus Hexan umkristallisiert und ergab 1,2 g (2E,4E)-3-Methyl-7-(5,5,8,8-tetramethyl-3-pentyl-5,6,7,8-tetrahydro-naphthalin-2-yl)-hepta-2,4-dien-6-insäure in weissen Kristallen, Schmelzpunkt 155-156°C.

In Analogie dazu ergab die Verseifung des (2Z,4E)-Esters 0,9 g der (2Z,4E)-Säure, Schmelzpunkt 165-166°C.

Beispiel 18

In Analogie zu Beispiel 17 wurde ausgehend von 1,1,4,4-Tetramethyl-6-hexyl-7-brom-1,2,3,4-tetrahydronaphthalin

die (2E,4E)-3-Methyl-7-(5,5,8,8-tetramethyl-3-hexyl-5,6,7,8-tetrahydro-naphthalin-2-yl)-hepta-2,4-dien-6-incarbonsäure erhalten.

Beispiel 19

Unter Argon legte man in 8ml abs. DMF 190 mg NaH (50% in Mineralöl) vor und gab bei 0° 1.43 g (2E,4E)-6-(Diethoxyphosphinyl)-3-methyl-2,4-hexadiensäure-diethylester zu. Nach beendeter $H_2$-Entwicklung liess man noch 30 Min. bei Raumtemperatur rühren, bevor man bei 0° 1.37 g (5RS,9SR,10RS)-2-Brom-3-butoxy-10-t-butyldimethylsilyloxy-5,9-dimethyl-6,7,8,9-tetrahydro-5,9-methano-5H-benzocyclohepten, gelöst in 4 ml abs. DMF, zutropfte. Nach 1 h bei Raumtemperatur goss man auf Eis, extrahierte mit Diethylether, wusch mit gesättigter NaCl- Lsg. und Wasser, trocknete über $Na_2SO_4$ und dampfte unter vermindertem Druck ein. Mitteldruck-Chromatographie an $SiO_2$ (Hexan/Ethylacetat=98.5/1.5) ergab, neben unpolareren Fraktionen, in welchen das 2Z-Isomere angereichert war, 1.33g (2E,4E,6E)-(5RS,9SR,10RS)-7-(3-Butoxy-10-t-butyldimethylsilyloxy-5,9-dimethyl-6,7,8,9-tetrahydro-5,9-methano-5H- benzocyclohepten-2-yl)-3-methyl-hepta-2,4,6-triensäure ethylester als gelben Schaum, welcher mit anderen Isomeren leicht verunreinigt war.

1.33 g der vorstehenden Verbindung wurden in 14 ml abs. THF gelöst und unter Argon über Nacht bei 35° mit 3.0 g $nBu_4NF$ behandelt. Man goss auf Eis, extrahierte mit Diethylether, wusch mit Wasser, trocknete und dampfte ein. Flash-Chromatographie und anschliessende Mitteldruckchromatographie an $SiO_2$ (Hexan/Ethylacetat=9/1) lieferte 370 mg (2E,4E,6E)-(5RS,9SR,10RS)-7-(3-Butoxy-10-hydroxy-5,9-dimethyl-6,7,8,9-tetrahydro- 5,9-methano-5H-benzocyclohepten-2-yl)-3-methyl-hepta-2,4,6-triensäure ethylester als gelben Schaum.

Dieser Ester wurde in 7 ml Ethanol/THF=1/1 gelöst und unter Argon mit 2.1 ml 2H NaOH versetzt. Man liess während 64 h bei Raumtemperatur reagieren und goss dann auf Eis/HCl/Ethylacetat. Die organische Phase wurde zweimal mit Wasser gewaschen, getrocknet und eingedampft. Umkristallisation aus Hexan/Ethylacetat=7/3 lieferte 153 mg (2E,4E,6E)-(5RS,9SR,10RS)-7-(3-Butoxy-10-hydroxy-5,9-dimethyl-6,7,8,9-tetrahydro- 5,9-methan-5H-benzocyclohepten-2-yl)-3-methyl-hepta-2,4,6-triensäure als gelbe Kristalle vom Smp. 176-178°.

Das als Ausgangsprodukt benötigte (5RS,9SR,10RS)-2-Brom-3-butoxy-10-t-butyldimethylsilyloxy-5,9-dimethyl-6,7,8,9-tetrahydro-5,9-methano-5H- benzocyclohepten wurde in Analogie zu dem in Beispiel 14 beschriebenen Verfahren aus den Synthesebausteinen p-Bromphenol, 1-Iodbutan und 2,6-Dimethylcyclohexanon hergestellt.

Beispiel 20

Unter Argon legte man 1.02 g NaH (50% in Mineralöl) in 60 ml abs. DMF vor und gab bei 0° 6.0 g 3-Brom-5,5,8,8-tetramethyl-5,6,7,8-tetrahydro-2-naphthol dazu. Man liess 1h bei 0° rühren und tropfte dann 2.28 ml Propargylbromid zu. Man entfernte das Kühlbad und liess 2 h nachreagieren. Man goss auf Eis, extrahierte mit Diethylether, wusch die organische Phase mit Wasser und gesättigter NaCl-Lsg., trocknete und entfernte das Lösungsmittel unter vermindertem Druck. Flash-Chromatographie an $SiO_2$ (Hexan/Ethylacetat=99/1) ergab 6.62 g 2-Brom-3-propargyloxy-5,5,8,8-tetramethyl-5,6,7,8-tetrahydro-2-naphthalin als weisse Kristalle vom Smp. 65-66°.

Nach Standardverfahren stellte man in abs. THF 24 mmol einer Lithium-diisopropylamid-Lösung her (ca. 0.25M) und tropfte dann bei 0° 6.62 g 2-Brom-3-propargyloxy-5,5,8,8-tetramethyl-5,6,7,8-tetrahydro-2-naphthalin, gelöst in 15 ml THF, zu. Nach 30 Min. wurden, immer noch bei 0°, 2.53 ml Ethyliodid und 2.5 ml DMPU (1,3-Dimethyl-3,4,5,6-tetrahydro-2-(1H)-pyrimidinon) zugegeben. Nach 4h goss man auf Eis, extrahierte mit Diethylether, wusch mit Wasser und gesättigter NaCl-Lsg., trocknete und dampfte unter vermindertem Druck ein. Flash-Chromatographie an $SiO_2$ (Hexan/Ethylacetat=98.5/1.5) lieferte 4.35 g ethyliertes Produkt.

Dieses wurde unter Argon in 50 ml abs. THF vorgelegt und bei -78° durch Behandeln mit 9.25 ml 1.6M nBuLi (Hexan) einem Metall-/Halogen-Austausch unterworfen. Nach 15 Min. spritzte man 2.8 ml abs. DMF zu und entfernte anschliessend das Kühlbad. Nach 1 h wurde das Reaktionsgemisch auf Eis gegossen, das Produkt mit Diethylether extrahiert, mit Wasser und gesättigter NaCl-Lsg. gewaschen, getrocknet und unter vermindertem Druck eingedampft. Flash-Chromatographie an $SiO_2$ (Hexan/Ethylacetat=96/4) ergab 2.17 g 2-Formyl-3-pent-2-ynyloxy-5,5,8,8-tetramethyl-5,6,7,8-tetrahydro-naphthalin als gelbliche Kristalle vom Smp. 90-91°.

Unter Argon legte man in 10 ml abs. DMF 193 mg NaH (55% in Mineralöl) vor und gab bei 0° 1.66 g Diethyl (4-carbethoxybenzyl) phosphonat zu und rührte anschliessend bei Raumtemperatur, bis die $H_2$-Entwicklung beendet war. Dann wurden bei 0° 1.08 g 2-Formyl-3-pent-2-ynyloxy-5,5,8,8-tetramethyl-5,6,7,8-tetrahydro-naphthalin ohne weiteres Lösungsmittel zugegeben. Nach 30 Min. bei Raumtemperatur extrahierte man mit Hexan und wusch zweimal mit Ethanol/$H_2O$=8/2. Trocknen, Eindampfen und direkte Kristallisation aus Hexan lieferte 1.45g (E)-4-[2-(3-Pent-2-ynyloxy-5,5,8,8-tetramethyl-5,6,7,8-tetrahydro-naphthalin-2-yl)-vinyl]- benzoesäure ethylester als gelbliche Kristalle vom Smp. 80-82°.

1.30 g (E)-4-[2-(3-Pent-2-ynyloxy-5,5,8,8-tetramethyl-5,6,7,8-tetrahydronaphthalin-2-yl)-vinyl]-benzoesäure ethylester wurden in 10 ml abs. Ethanol/THF=1/1 vorgelegt und unter Argon mit 4.9 ml 3N NaOH versetzt. Nach 40 h bei

Raumtemperatur goss man auf Eis und extrahierte mit Ethylacetat. Die organische Phase wurde mit Wasser und gesättigter NaCl-Lsg. gewaschen, getrocknet und eingedampft. Kristallisation aus Hexan/Ethylacetat lieferte 1.07 g (E)-4-[2-(3-Pent-2-ynyloxy-5,5,8,8-tetramethyl-5,6,7,8-teträhydro-naphthalin-2-yl)-vinyl]-benzoesäure als beige Kristalle vom Smp. 144-145°.

Beispiel 21

Unter Argon legte man 212 mg NaH (50% in Mineralöl) in 8 ml abs. DMF vor und tropfte bei 0° 1.50 g (2E,4E)-6-(Diethoxyphosphinyl)-3-methyl-2,4-hexadiensäure diethylester unverdünnt zu. Nach beendeter $H_2$-Entwicklung gab man 1.10 g 2-Formyl-3-pent-2-ynyloxy-5,5,8,8-tetramethyl-5,6,7,8-tetrahydro-naphthalin (siehe Bsp. 20) zu und liess 1.5 h bei Raumtemperatur nachreagieren. Das Reaktionsprodukt wurde auf Eis gegossen, mit Hexan extrahiert, zweimal mit Ethanol/$H_2O$=8/2 getrocknet und eingedampft. Flash-Chromatographie an $SiO_2$ (Hexan/Ethylacetat= 98.5/1.5) lieferte, neben 412 mg 2Z-Isomeren in den unpolareren Fraktionen, 1026 mg (2E,4E,6E)-3-Methyl-7-(3-pent-2-ynyloxy-5,5,8,8-tetramethyl-5,6,7,8-tetrahydro-naphthalin-2-yl)-hepta-2,4,6-triensäure-ethylester als gelbes Oel.

1.02g (2E,4E,6E)-3-Methyl-7-(3-pent-2-ynyloxy-5,5,8,8-tetramethyl-5,6,7,8-tetrahydro-naphthalin-2-yl)-hepta-2,4,6-triensäure- ethylester wurden in 8 ml abs. THF/Ethanol (1/1) vorgelegt und unter Argon mit 3.9 ml 3H NaOH versetzt. Man rührte 2 Tage bei Raumtemperatur und 3 h bei 40°. Dann goss man auf Eis/HCl, extrahierte mit Diethylether, wusch mit Wasser, trocknete und dampfte ein. Kristallisation aus Diethylether lieferte 644 mg (2E,4E,6E)-3-Methyl-7-(3-pent-2-ynyloxy-5,5,8,8-tetramethyl-5,6,7,8-tetrahydro-naphthalin-2-yl)-hepta-2,4,6-triensäure als gelbe Kristalle vom Smp. 208-209°.

Beispiel 22

In Analogie zu Beispiel 20 wurde mit Allylbromid als Elektrophil in der ersten Stufe, die

(E)-4-[2-(3-Allyloxy-5,5,8,8-tetramethyl-5,6,7,8-tetrahydro-naphthalin-2-yl)-vinyl]-benzoesäure vom Smp. 191-192° und mit Crotylbromid als Elektrophil in der ersten Stufe, die
4-[(E)-2-((E)-3-But-2-enyloxy-5,5,8,8-tetramethyl-5,6,7,8-tetrahydronaphthalin-2-yl)-vinyl]-benzoesäure vom Smp. 178-180° hergestellt.

Beispiel 23

In Analogie zu Beispiel 21 wurde mit Allylbromid als Elektrophil in der ersten Stufe (vgl. auch Bsp. 20), die

(2E,4E,6E)-7-(3-Allyloxy-5,5,8,8-tetramethyl-5,6,7,8-tetrahydro-naphthalin-2-yl)-3-methyl-hepta-2,4,6-triensäure vom Smp. 193-194°;
und mit Crotylbromid als Elektrophil in der ersten Stufe die
(2E,4E,6E)-7-[(E)-3-But-2-enyloxy-5,5,8,8-tetramethyl-5,6,7,8-tetrahydronaphthalin-2-yl)-3-methyl-hepta-2,4,6-triensäure vom Smp. 196-198° hergestellt.

Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 20 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| | Total 120 |

Das sprühgetrocknete Pulver, das auf dem Wirkstoff, Gelatine und mikrokristalliner Cellulose basiert und eine mittlere Korngrösse des Wirkstoffes von <1 µ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | mg/Tablette |
|---|---|
| 1. Verbindung I als feingemahlenes Pulver | 20 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| | Total 320 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | mg/Kapsel |
|---|---|
| 1. Verbindung I | 5 |
| 2. Triglycerid | 450 |
| | Total 455 |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 5 mg Wirkstoff verarbeitet.

Beispiel D

Eine Lotion kann wie folgt hergestellt werden:

| Bestandteile | |
|---|---|
| 1. Verbindung I, feingemahlen | 1,0 g |

(fortgesetzt)

| Bestandteile | |
|---|---|
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

Beispiel E

Eine Crème kann aus den nachstehend aufgeführten Inhaltsstoffen in an sich bekannter Weise hergestellt werden:

| | Gew.-% |
|---|---|
| Verbindung der Formel I | 0,1-5 |
| Cetylalkohol | 5,25-8,75 |
| Arlacel 165 (Glyceryl/PEG 100-stearat) | 3,75-6,25 |
| Miglyol 818 (Capryl-/Caprin-/Linolsäure triglycerid) | 11,25-18,75 |
| Sorbit-Lösung | 3,75-6,25 |
| EDTA-$Na_2$ | 0,075-0,125 |
| Carbopol 934P (Carbomer 934P) | 0,15-0,25 |
| butyliertes Hydroxyanisol | 0,0375-0,0625 |
| Methylparaben | 0,135-0,225 |
| Propylparaben | 0,0375-0,0625 |
| NaOH (10% solution) | 0,15-0,25 |
| Wasser q.s. | 100,00 |

Beispiel F

Ein Gel kann aus den nachstehend aufgeführten Inhaltsstoffen in an sich bekannter Weise hergestellt werden:

| | Gew.-% |
|---|---|
| Verbindung der Formel I | 0,1-5 |
| Pluronic L 101 (Poloxamer 331) | 10,00 |
| Aerosil 200 (Siliciumdioxid) | 8,00 |
| PCL Liquid (Fettsäureester | 15,00 |
| Cetiol V (Decyloleat) | 20,00 |
| Neobee Oil (Triglycerid mittlerer Kettenlänge) | 15,00 |

(fortgesetzt)

| | Gew.-% |
|---|---|
| Euhanol G (Octyldodecanol), q.s. | 100,00 |

Durch Variation der Verhältnisse zwischen den Hilfsstoffen der Beispiele E und F können die physikalischen Eigenschaften der Präparate verändert werden.

**Patentansprüche**

1. Verwendung von Verbindungen der Formel

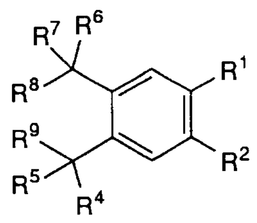

I

worin

$R^1$ einen Rest der Formeln

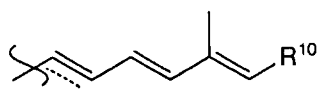

(a)

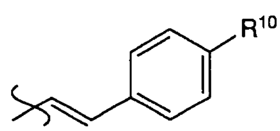

(b)

| | |
|---|---|
| $R^2$ | $C_{2-8}$-Alkanoyl, $C_{2-8}$Alkyl, $C_{2-8}$-Alkenyl, $C_{2-8}$-Alkinyl oder $-OCH_2R^3$; |
| $R^3$ | Wasserstoff oder $C_{1-6}$-Alkyl, $C_{2-6}$-Alkenyl oder $C_{2-6}$-Alkinyl; |
| $R^4$ bis $R^9$ | unabhängig voneinander Wasserstoff oder $C_{1-5}$-Alkyl bedeuten; oder |
| $R^8$ und $R^9$ | zusammengenommen $(CR^aR^b)_n$, $R^a$ und $R^8$ Wasserstoff oder $C_{1-5}$-Alkyl, n 1, 2 oder 3 und $R^4$ bis $R^7$ dasselbe wie oben bedeuten; oder |
| $R^8$ und $R^9$ | zusammengenommen $(CR^aR^b)_n$, und $R^4$ und $R^6$ zusammengenommen Methylen oder Aethylen, die durch Hydroxy substituiert sein können, und $R^a$, $R^b$, $R^5$, $R^7$ und n dasselbe wie oben bedeuten; |
| $R^{10}$ | Carboxyl, $C_{1-6}$-Alkoxycarbonyl oder mono- oder di-($C_{1-6}$-Alkyl)carbamoyl darstellen, und die punktierte Bindung in der Formel (a) fakultativ ist; |

und pharmazeutisch anwendbare Salze von Carbonsäuren der Formel I bei der Herstellung von pharmazeutischen Präparaten zur Therapie und Prophylaxe von licht- und altersbedingten Schäden der Haut sowie zur Förderung der Wundheilung,

2. Verwendung gemäss Anspruch 1 von Verbindungen der Formel I, in denen $R^1$ ein Rest (a) ist.

3. Verwendung gemäss Anspruch 1 von Verbindungen der Formel I, in denen $R^1$ ein Rest (b) ist.

4. Verwendung gemäss Anspruch 1-3 von Verbindungen der Formel I, in denen $R^2$ $C_{2-8}$-Alkyl oder $-OCH_2R^3$ und $R^3$ $C_{1-6}$-Alkyl ist.

5. Verwendung gemäss Anspruch 1-3 von Verbindungen der Formel I, in denen $R^2$ $C_{2-8}$-n-Alkanoyl ist.

6. Verwendung gemäss Anspruch 1-3 von Verbindungen der Formel I, in denen $R^2$ $-OCH_2R^3$ und $R^3$ $C_{2-6}$-Alkenyl oder $C_{2-6}$-Alkinyl ist.

7. Verwendung gemäss Anspruch 1-6 von Verbindungen der Formel I, in denen $R^4$ bis $R^7$ unabhängig voneinander Wasserstoff oder $C_{1-5}$-Alkyl und $R^8$ und $R^9$ zusammengenommen $(CR^aR^b)_n$ bedeuten.

8. Verwendung gemäss Anspruch 7 von Verbindungen der Formel I, in denen $R^8$ und $R^9$ zusammengenommen Methylen oder Aethylen bedeuten.

9. Verwendung gemäss Anspruch 1 der Verbindungen

Aethyl p-[(E)-2-(3-hexyl-5,6,7,8-tetrahydro-5,5,8,8-tetramethyl-2-naphthyl)vinyl]benzoat,
p-[(E)-2-(3-Hexyl-5,6,7,8-tetrahydro-5,5,8,8-tetramethyl-2-naphthyl)-vinyl]benzoesäure,
(E)-4-[2-(3-Pentyl-5,5,8,8-tetramethyl-5,6,7,8-tetrahydro-naphthalin-2-yl)vinyl]benzoesäure,
(E)-4-[2-(3-Butyl-5,5,8,8-tetramethyl-5,6,7,8-tetrahydro-naphthalin-2-yl)vinyl]benzoesäure,
(2Z,4E,6E)-7-(3-Hexyl-5,5,8,8-tetramethyl-5,6,7,8-tetrahydro-naphthalin-2-yl)-3-methyl-hepta-2,4,6-triencarbonsäuremethylester,
(all-E)-7-(3-Hexyl-5,5,8,8-tetramethyl-5,6,7,8-tetrahydro-naphthalin-2-yl)-3-methyl-hepta-2,4,6-triencarbonsäure,
(2E,4E,6E)-7-(3-Pentyl-5,5,8,8-tetramethyl-5,6,7,8-tetrahydro-naphthalin-2-yl)-3-methyl-hepta-2,4,6-triencarbonsäure,
(2Z,4E,6E)-7-(3-Pentyl-5,5,8,8-tetramethyl-5,6,7,8-tetrahydro-naphthalin-2-yl)-3-methyl-hepta-2,4,6-triencarbonsäure,
(E)-4-[2-(5,5,8,8-Tetramethyl-3-propoxy-5,6,7,8-tetrahydro-naphthalin-2-yl)vinyl]benzoesäureäthylester,
(E)-4-[2-(5,5,8,8-Tetramethyl-3-propoxy-5,6,7,8-tetrahydro-naphthalin-2-yl)vinyl]benzoesäure,
(E)-4-[2-(5,5,8,8-Tetramethyl-3-pentoxy-5,6,7,8-tetrahydro-naphthalin-2-yl)vinyl]benzoesäure,
(2E,4E,6E)-3-Methyl-7-(5,5,8,8-tetramethyl-3-propoxy-5,6,7,8-tetrahydro-naphthalin-2-yl)-hepta-2,4,6-triencarbonsäureäthylester,
(2E,4E,6E)-3-Methyl-7-(5,5,8,8-tetramethyl-3-propoxy-5,6,7,8-tetrahydro-naphthalin-2-yl)-hepta-2,4,6-triencarbonsäure,
(2Z,4E,6E)-3-Methyl-7-(5,5,8,8-tetramethyl-3-propoxy-5,6,7,8-tetrahydro-naphthalin-2-yl)-hepta-2,4,6-triencarbonsäure,
2E,4E,6E)-3-Methyl-7-(5,5,8,8-tetramethyl-3-pentoxy-5,6,7,8-tetrahydro-naphthalin-2-yl)-hepta-2,4,6-triencarbonsäure,
(2Z,4E,6E)-3-Methyl-7-(5,5,8,8-tetramethyl-3-pentoxy-5,6,7,8-tetrahydro-naphthalin-2-yl)-hepta-2,4,6-triencarbonsäure,
(E)-4-[2-(7,7-Dimethyl-3-octyl-6,7,8,9-tetrahydro-5H-benzocyclohepten-2-yl)vinyl]benzoesäureäthylester,
(E)-4-[2-(7,7-Dimethyl-3-octyl-6,7,8,9-tetrahydro-5H-benzocyclohepten-2-yl)vinyl]benzoesäure,
rac-(E)-4-[2-(5,7,7-Trimethyl-3-octyl-6,7,8,9-tetrahydro-5H-benzocyclohepten-2-yl)vinyl]benzoesäure,
(E)-4-[2-(3-Butyl-5,5-dimethyl-6,7,8,9-tetrahydro-5H-benzocyclohepten-2-yl)vinyl]benzoesäure,
(E)-4-[2-(3-Hexyl-7,7-dimethyl-indan-2-yl)vinyl]benzoesäure,
(E)-4-[2-(3-Hexyl-5,5-dimethyl-6,7,8,9-tetrahydro-5H-benzocyclohepten-2-yl)vinyl]benzoesäure,
(E)-4-[2-(3-Butyl-9,9-dimethyl-6,7,8,9-tetrahydro-5H-benzocyclohepten-2-yl)vinyl]benzoesäure,
(E)-4-[2-[(5RS,9SR,10RS)-3-Heptyloxy-10-hydroxy-5,9-dimethyl-6,7,8,9-tetrahydro-5,9-methano-5H-benzocyclohepten-2-yl]vinyl]benzoesäureäthylester,
(E)-4-[2-[(5RS,9SR,10RS)-3-Heptyloxy-10-hydroxy-5,9-dimethyl-6,7,8,9-tetrahydro-5,9-methano-5H-benzocyclohepten-2-yl]vinyl]benzoesäure,
(E)-4-[2-[(5RS,9SR,10RS)-3-Butoxy-10-hydroxy-5,9-dimethyl-6,7,8,9-tetrahydro-5,9-methano-5H-benzocyclohepten-2-yl]vinyl]benzoesäureäthylester,
(E)-4-[2-[(5RS,9SR,10RS)-3-Butoxy-10-hydroxy-5,9-dimethyl-6,7,8,9-tetrahydro-5,9-methano-5H-benzocyclohepten-2-yl]vinyl]benzoesäure.

10. Verwendung gemäss Anspruch 1 der Verbindungen

(2E,4E)-3-Methyl-7-(5,5,8,8-tetramethyl-3-pentyl-5,6,7,8-tetrahydro-naphthalin-2-yl)-hepta-2,4-dien-6-in-säureäthylester,

(2E,4E)-3-Methyl-7-(5,5,8,8-tetramethyl-3-pentyl-5,6,7,8-tetrahydro-naphthalin-2-yl)-hepta-2,4-dien-6-insäure,
(2E,4E)-3-Methyl-7-(5,5,8,8-tetramethyl-3-hexyl-5,6,7,8-tetrahydro-naphthalin-2-yl)-hepta-2,4-dien-6-incarbonsäure,
(2E,4E,6E)-(5RS,9SR,10RS)-7-(3-Butoxy-10-t-butyldimethylsilyloxy-5,9-dimethyl-6,7,8,9-tetrahydro-5,9-methano-5H-benzocyclohepten-2-yl)-3-methyl-hepta-2,4,6-triensäure ethylester,
(2E,4E,6E)-(5RS,9SR,10RS)-7-(3-Butoxy-10-hydroxy-5,9-dimethyl-6,7,8,9-tetrahydro-5,9-methano-5H-benzocyclohepten-2-yl)-3-methyl-hepta-2,4,6-triensäure ethylester,
(2E,4E,6E)-(5RS,9SR,10RS)-7-(3-Butoxy-10-hydroxy-5,9-dimethyl-6,7,8,9-tetrahydro-5,9-methano-5H-benzocyclohepten-2-yl)-3-methyl-hepta-2,4,6-triensäure.

11. Verwendung gemäss Anspruch 1 der Verbindung

(E)-4-[2-(3-Hexanoyl-5,5,8,8-tetramethyl-5,6,7,8-tetrahydro-naphthalin-2-yl)vinyl]benzoesäure.

12. Verwendung gemäss Anspruch 1 der Verbindungen

2-Brom-3-propargyloxy-5,5,8,8-tetramethyl-5,6,7,8-tetrahydro-2-naphthalin,
2-Formyl-3-pent-2-ynyloxy-5,5,8,8-tetramethyl-5,6,7,8-tetrahydro-naphthalin,
(E)-4-[2-(3-Pent-2-ynyloxy-5,5,8,8-tetramethyl-5,6,7,8-tetrahydro-naphthalin-2-yl)-vinyl]-benzoesäure ethylester,
(E)-4-[2-(3-Pent-2-ynyloxy-5,5,8,8-tetramethyl-5,6,7,8-tetrahydro-naphthalin-2-yl)-vinyl]-benzoesäure,
(2E,4E,6E)-3-Methyl-7-(3-pent-2-ynyloxy-5,5,8,8-tetramethyl-5,6,7,8-tetrahydro-naphthalin-2-yl)-hepta-2,4,6-triensäure-ethylester,
(2E,4E,6E)-3-Methyl-7-(3-pent-2-ynyloxy-5,5,8,8-tetramethyl-5,6,7,8-tetrahydro-naphthalin-2-yl)-hepta-2,4,6-triensäure,
(E)-4-[2-(3-Allyloxy-5,5,8,8-tetramethyl-5,6,7,8-tetrahydro-naphthalin-2-yl)-vinyl]-benzoesäure,
4-[(E)-2-((E)-3-But-2-enyloxy-5,5,8,8-tetramethyl-5,6,7,8-tetrahydro-naphthalin-2-yl)-vinyl]-benzoesäure,
(2E,4E,6E)-7-(3-Allyloxy-5,5,8,8-tetramethyl-5,6,7,8-tetrahydro-naphthalin-2-yl)-3-methyl-hepta-2,4,6-triensäure,
(2E,4E,6E)-7-[(E)-3-But-2-enyloxy-5,5,8,8-tetramethyl-5,6,7,8-tetrahydro-naphthalin-2-yl)-3-methyl-hepta-2,4,6-triensäure.

13. Verwendung gemäss Anspruch 1 von Verbindungen der Formel I, in denen $R^2$ $C_{2-8}$-Alkyl oder $C_{1-7}$-Alkoxy darstellt.

14. Verbindungen der Formel I gemäss Definition in Anspruch 1, in denen $R^2$ $C_{2-8}$-n-Alkanoyl ist.

15. Verbindungen der Formel I gemäss Definition in Anspruch 1, in denen $R^2$ $-OCH_2R^3$ und $R^3$ $C_{2-6}$-Alkenyl oder $C_{2-6}$-Alkinyl ist.

## Claims

1. The use of compounds of the formula

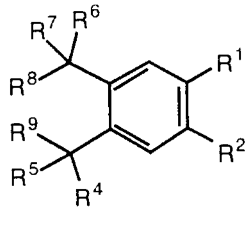

I

wherein

$R^1$ signifies a residue of the formula

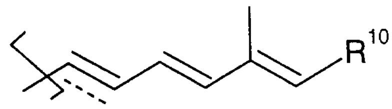

(a)

or

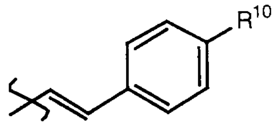

(b)

$R^2$ signifies $C_{2-8}$-alkanoyl, $C_{2-8}$-alkyl, $C_{2-8}$-alkenyl, $C_{2-8}$-alkynyl or $-OCH_2R^3$;
$R^3$ signifies hydrogen or $C_{1-6}$-alkyl, $C_{2-6}$-alkenyl or $C_{2-6}$-alkynyl;
$R^4$ to $R^9$ each independently signify hydrogen or $C_{1-5}$-alkyl; or
$R^8$ and $R^9$ together signify $(CR^aR^b)_n$, $R^a$ and $R^b$ (sic) signify hydrogen or $C_{1-5}$-alkyl, n signifies 1, 2 or 3 and $R^4$ to $R^7$ signify the same as above; or
$R^8$ and $R^9$ together signify $(CR^aR^b)_n$ and $R^4$ and $R^6$ together signify methylene or ethylene, which can be substituted by hydroxy, and $R^a$,$R^b$, $R^5$, $R^7$ and n signify the same as above;
$R^{10}$ signifies carboxyl, $C_{1-6}$-alkoxycarbonyl or mono- or di-($C_{1-6}$-alkyl)carbamoyl and the dotted bond in formula (a) is optional;

and pharmaceutically acceptable salts of carboxylic acids of formula I in the production of pharmaceutical preparations for the therapy and prophylaxis of light- and age-damaged skin and for the promotion of wound healing

2. The use according to claim 1 of compounds of formula I in which $R^1$ is a residue (a).

3. The use according to claim 1 of compounds of formula I in which $R^1$ is a residue (b).

4. The use according to claim 1-3 of compounds of formula I in which $R^2$ is $C_{2-8}$-alkyl or $-OCH_2R^3$ and $R^3$ is $C_{1-6}$-alkyl.

5. The use according to claim 1-3 of compounds of formula I in which $R^2$ is $C_{2-8}$-n-alkanoyl.

6. The use according to claim 1-3 of compounds of formula I in which $R^2$ is $-OCH_2R^3$ and $R^3$ is $C_{2-6}$-alkenyl or $C_{2-6}$-alkynyl.

7. The use according to claim 1-6 of compounds of formula I in which $R^4$ to $R^7$ each independently signify hydrogen or $C_{1-5}$-alkyl and $R^8$ and $R^9$ together signify $(CR^aR^b)_n$.

8. The use according to claim 7 of compounds of formula I in which $R^8$ and $R^9$ together signify methylene or ethylene.

9. The use according to claim 1 of the compounds

ethyl p-[(E)-2-(3-hexyl-5,6,7,8-tetrahydro-5,5,8,8-tetramethyl-2-naphthyl)vinyl]benzoate,
p-[(E)-2-(3-hexyl-5,6,7,8-tetrahydro-5,5,8,8-tetramethyl-2-naphthyl)-vinyl]benzoic acid,
(E)-4-[2-(3-pentyl-5,5,8,8-tetramethyl-5,6,7,8-tetrahydro-naphthalen-2-yl)vinyl]benzoic acid,
(E)-4-[2-(3-butyl-5,5,8,8-tetramethyl-5,6,7,8-tetrahydro-naphthalen-2-yl)vinyl]benzoic acid,
methyl (2Z,4E,6E)-7-(3-hexyl-5,5,8,8-tetramethyl-5,6,7,8-tetrahydro-naphthalen-2-yl)-3-methyl-hepta-2,4,6-trienecarboxylate,
(all-E)-7-(3-hexyl-5,5,8,8-tetramethyl-5,6,7,8-tetrahydro-naphthalen-2-yl)-3-methyl-hepta-2,4,6-trienecarboxylic acid,
(2E,4E,6E)-7-(3-pentyl-5,5,8,8-tetramethyl-5,6,7,8-tetrahydro-naphthalen-2-yl)-3-methyl-hepta-2,4,6-trienecarboxylic acid,
(2Z,4E,6E)-7-(3-pentyl-5,5,8,8-tetramethyl-5,6,7,8-tetrahydro-naphthalen-2-yl)-3-methyl-hepta-2,4,6-trienecarboxylic acid,
ethyl (E)-4-[2-(5,5,8,8-tetramethyl-3-propoxy-5,6,7,8-tetrahydro-naphthalen-2-yl)vinyl]benzoate,
(E)-4-[2-(5,5,8,8-tetramethyl-3-propoxy-5,6,7,8-tetrahydro-naphthalen-2-yl)vinyl]benzoic acid,
(E)-4-[2-(5,5,8,8-tetramethyl-3-pentoxy-5,6,7,8-tetrahydro-naphthalen-2-yl)vinyl]benzoic acid,
ethyl (2E,4E,6E)-3-methyl-7-(5,5,8,8-tetramethyl-3-propoxy-5,6,7,8-tetrahydro-naphthalen-2-yl)-hepta-2,4,6-trienecarboxylate,
(2E,4E,6E)-3-methyl-7-(5,5,8,8-tetramethyl-3-propoxy-5,6,7,8-tetrahydro-naphthalen-2-yl)-hepta-2,4,6-trienecarboxylic acid,
(2Z,4E,6E)-3-methyl-7-(5,5,8,8-tetramethyl-3-propoxy-5,6,7,8-tetrahydro-naphthalen-2-yl)-hepta-2,4,6-trienecarboxylic acid,
2E,4E,6E)-3-methyl-7-(5,5,8,8-tetramethyl-3-pentoxy-5,6,7,8-tetrahydro-naphthalen-2-yl)-hepta-2,4,6-trienecarboxylic acid,
(2Z,4E,6E)-3-methyl-7-(5,5,8,8-tetramethyl-3-pentoxy-5,6,7,8-tetrahydro-naphthalen-2-yl)-hepta-2,4,6-trienecarboxylic acid,
ethyl (E)-4-[2-(7,7-dimethyl-3-octyl-6,7,8,9-tetrahydro-5H-benzocyclo-hepten-2-yl)vinyl]benzoate,
(E)-4-[2-(7,7-dimethyl-3-octyl-6,7,8,9-tetrahydro-5H-benzocyclohepten-2-yl)vinyl)benzoic acid,
rac-(E)-4-[2-(5,7,7-trmethyl-3-octyl-6,7,8,9-tetrahydro-5H-benzocyclo-hepten-2-yl)vinyl]benzoic acid,
(E)-4-[2-(3-butyl-5,5-dimethyl-6,7,8,9-tetrahydro-5H-benzocyclohepten-2-yl)vinyl]benzoic acid,
(E)-4-[2-(3-hexyl-7,7-dimethyl-indan-2-yl)vinyl]benzoic acid,
(E)-4-[2-(3-hexyl-5,5-dimethyl-6,7,8,9-tetrahydro-5H-benzocyclo-hepten-2-yl)vinyl]benzoic acid,
(E)-4-[2-(3-butyl-9,9-dimethyl-6,7,8,9-tetrahydro-5H-benzocyclohepten-2-yl)vinyl]benzoic acid,
ethyl (E)-4-[2-[(5RS,9SR,10RS)-3-heptyloxy-10-hydroxy-5,9-dimethyl-6,7,8,9-tetrahydro-5,9-methano-5H-benzocyclohepten-2-yl]vinyl]benzoate,
(E)-4-[2-[(5RS,9SR,10RS)-3-heptyloxy-10-hydroxy-5,9-dimethyl-6,7,8,9-tetrahydro-5,9-methano-5H-benzocyclohepten-2-yl]vinyl]benzoic acid,
ethyl (E)-4-[2-[(5RS,9SR,10RS)-3-butoxy-10-hydroxy-5,9-dimethyl-6,7,8,9-tetrahydro-5,9-methano-5H-benzocyclohepten-2-yl]vinyl]benzoate,
(E)-4-[2-[(5RS,9SR,10RS)-3-butoxy-10-hydroxy-5,9-dimethyl-6,7,8,9-tetrahydro-5,9-methano-5H-benzocyclohepten-2-yl]vinyl]benzoic acid.

**10.** The use according to claim 1 of the compounds

ethyl (2E,4E)-3-methyl-7-(5,5,8,8-tetramethyl-3-pentyl-5,6,7,8-tetrahydronaphthalen-2-yl)-hepta-2,4-dien-6-ynoate,
(2E,4E)-3-methyl-7-(5,5,8,8-tetramethyl-3-pentyl-5,6,7,8-tetrahydro-naphthalen-2-yl)-hepta-2,4-dien-6-ynoic acid,
(2E,4E)-3-methyl-7-(5,5,8,8-tetramethyl-3-hexyl-5,6,7,8-tetrahydro-naphthalen-2-yl)-hepta-2,4-dien-6-ynoic acid,
ethyl (2E,4E,6E)-(5RS,9SR,10RS)-7-(3-butoxy-10-t-butyl dimethy-l silyloxy-5,9-dimethyl-6,7,8,9-tetrahydro-5,9-methano-5H-benzocyclohepten-2-yl)-3-methyl-hepta-2,4,6-trienoate,
ethyl (2E,4E,6E)-(5RS,9SR,10RS)-7-(3-butoxy-10-hydroxy-5,9-dimethyl-6,7,8,9-tetrahydro-5,9-methano-5H-benzocyclohepten-2-yl)-3-methyl-hepta-2,4,6-trienoate,
(2E,4E,6E)-(5RS,9SR,10RS)-7-(3-butoxy-10-hydroxy-5,9-dimethyl-6,7,8,9-tetrahydro-5,9-methano-5H-benzocyclohepten-2-yl)-3-methyl-hepta-2,4,6-trienoic acid.

**11.** The use according to claim 1 of the compound

(E)-4-[2-(3-hexanoyl-5,5,8,8-tetramethyl-5,6,7,8-tetrahydro-naphthalen-2-yl)vinyl]benzoic acid.

**12.** The use according to claim 1 of the compounds

2-bromo-3-propargyloxy-5,5,8,8-tetramethyl-5,6,7,8-tetrahydro-2-naphthalene,
2-formyl-3-pent-2-ynyloxy-5,5,8,8-tetramethyl-5,6,7,8-tetrahydro-naphthalene,
ethyl (E)-4-[2-(3-pent-2-ynyloxy-5,5,8,8-tetramethyl-5,6,7,8-tetrahydro-naphthalen-2-yl)-vinyl]-benzoate,
(E)-4-[2-(3-pent-2-ynyloxy-5,5,8,8-tetramethyl-5,6,7,8-tetrahydro-naphthalen-2-yl)-vinyl]-benzoic acid,
ethyl (2E,4E,6E)-3-methyl-7-(3-pent-2-ynyloxy-5,5,8,8-tetramethyl-5,6,7,8-tetrahydro-naphthalen-2-yl)-hepta-2,4,6-trienoate,
(2E,4E,6E)-3-methyl-7-(3-pent-2-ynyloxy-5,5,8,8-tetramethyl-5,6,7,8-tetrahydro-naphthalen-2-yl)-hepta-2,4,6-trienoic acid,
(E)-4-[2-(3-allyloxy-5,5,8,8-tetramethyl-5,6,7,8-tetrahydro-naphthalen-2-yl)-vinyl]-benzoic acid,
4-[(E)-2-((E)-3-but-2-enyloxy-5,5,8,8-tetramethyl-5,6,7,8-tetrahydro-naphthalen-2-yl)-vinyl]-benzoic acid,
(2E,4E,6E)-7-(3-allyloxy-5,5,8,8-tetramethyl-5,6,7,8-tetrahydro-naphthalen-2-yl)-3-methyl-hepta-2,4,6-trienoic acid,
(2E,4E,6E)-7-[(E)-3-but-2-enyloxy-5,5,8,8-tetramethyl-5,6,7,8-tetrahydro-naphthalen-2-yl)-3-methyl-hepta-2,4,6-trienoic acid.

**13.** The use according to claim 1 of compounds of formula I in which $R^2$ is $C_{2-8}$-alkyl or $C_{1-7}$-alkoxy.

**14.** Compounds of formula I according to the definition in claim 1, in which $R^2$ is $C_{2-8}$-n-alkanoyl.

**15.** Compounds of formula I according to the definition in claim 1, in which $R^2$ is $-OCH_2R^3$ and $R_3$ is $C_{2-6}$-alkenyl or $C_{2-6}$-alkynyl.

**Revendications**

**1.** Utilisation de composés de formule

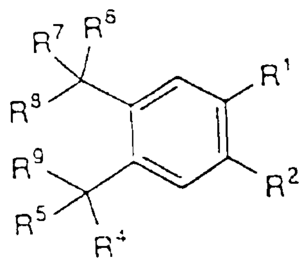

(I)

dans laquelle

- $R^1$ est un radical ayant les formules

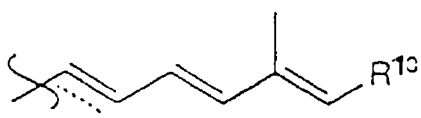

(a)

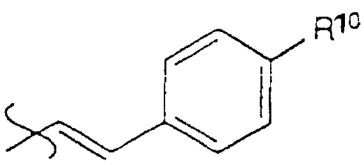

(b)

- $R^2$ est un radical alcanoyle en $C_{2-8}$, alkyle en $C_{2-8}$, alcényle en $C_{2-8}$, alcynyle en $C_{2-8}$ ou $-OCH_2R^3$ ;
- $R^3$ est un atome d'hydrogène ou un radical alkyle en $C_{1-6}$, alcényle en $C_{2-6}$ ou alcynyle en $C_{2-6}$ ;
- $R^4$ à $R^9$ représentent chacun indépendamment de l'autre un atome d'hydrogène ou un radical alkyle en $C_1$-$C_5$ ; ou bien
- $R^8$ et $R^9$, pris ensemble, représentent $(CR^aR^b)_n$, $R^a$ et $R^8$ sont des atomes d'hydrogène ou des groupes alkyle en $C_{1-5}$, n vaut 1, 2 ou 3, et $R^4$ à $R^7$ ont les mêmes significations que ci-dessus,
- $R^8$ et $R^9$, pris ensemble, représentent $(CR^aR^b)_n$, et $R^4$ et $R^6$, pris ensemble, représentent des radicaux méthylène ou éthylène pouvant être substitués par des substituants hydroxy, et $R^a$, $R^b$, $R^5$, $R^7$ et n ont les mêmes

significations que ci-dessus ;

- $R^{10}$ est un radical carboxyle, (alcoxy en $C_{1-6}$)carbonyle, ou mono- ou di-(alkyle en $C_{1-6}$)carbamoyle, et la liaison en pointillés se trouvant dans la formule (a) est facultative ;
- et des sels utilisables d'un point de vue pharmaceutique d'acides carboxyliques de formule I,

pour fabriquer des préparations pharmaceutiques destinées au traitement et à la prophylaxie des lésions cutanées provoquées par la lumière et l'âge, ainsi qu'à l'avancement de la cicatrisation.

2. Utilisation selon la revendication 1 de composés de formule I dans laquelle $R^1$ est un radical (a).

3. Utilisation selon la revendication 1 de composés de formule I dans laquelle $R^1$ est un radical (b).

4. Utilisation selon les revendications 1 à 3 de composés de formule I, dans laquelle $R^2$ est un radical alkyle en $C_{2-8}$ ou $-OCH_2R^3$, et $R^3$ est un radical alkyle en $C_{1-6}$.

5. Utilisation selon les revendications 1 à 3 de composés de formule I dans laquelle $R^2$ est un radical n-alcanoyle en $C_{2-8}$.

6. Utilisation selon les revendications 1 à 3 de composés de formule I dans laquelle $R^2$ est $-OCH_2R^3$, et $R^3$ est un radical alcényle en $C_{2-6}$ ou alcynyle en $C_{2-6}$.

7. Utilisation selon les revendications 1 à 6 de composés de formule I dans laquelle $R^4$ à $R^7$ représentent indépendamment les uns des autres des atomes d'hydrogène ou des radicaux alkyle en $C_{1-5}$, et $R^8$ et $R^9$, pris ensemble, représentent $(CR^aR^b)_n$.

8. Utilisation selon la revendication 7 de composés de formule I dans laquelle $R^8$ et $R^9$, pris ensemble, forment des radicaux méthylène ou éthylène.

9. Utilisation selon la revendication 1 des composés suivants :

- p-[(E)-2-(3-hexyl-5,6,7,8-tétrahydro-5,5,8,8-tétraméthyl-2-naphtyl)-vinyl]-benzoate d'éthyle,
- acide p-[(E)-2-(3-hexyl-5,6,7,8-tétrahydro-5,5,8,8-tétraméthyl-2-naphtyl)-vinyl]benzoïque,
- acide (E)-4-[2-(3-pentyl-5,5,8,8-tétraméthyl-5,6,7,8-tétrahydronaphtalène-2-yl)vinyl]benzoïque,
- acide (E)-4-[2-(3-butyl-5,5,8,8-tétraméthyl-5,6,7,8-tétrahydronaphtalène-2-yl)vinyl]benzoïque,
- ester méthylique de l'acide (2Z,4E,6E)-7-(3-hexyl-5,5,8,8-tétraméthyl-5,6, 7,8-tétrahydronaphtalène-2-yl)-3-méthyl-hepta-2,4,6-triènecarboxylique,
- acide (all-E)-7-(3-hexyl-5,5,8,8-tétraméthyl-5,6,7,8-tétrahydronaphtalène-2-yl)-3-méthyl-hepta-2,4,6-triènecarboxylique,
- acide (2E,4E,6E)-7-(3-pentyl-5,5,8,8-tétraméthyl-5,6,7,8-tétrahydro-naphtalène-2-yl)-3-méthyl-hepta-2,4,6-triènecarboxylique,
- acide (2Z,4E,6E)-7-(3-pentyl-5,5,8,8-tétraméthyl-5,6,7,8-tétrahydro-naphtalène-2-yl)-3-méthyl-hepta-2,4,6-triènecarboxylique,
- ester éthylique de l'acide (E)-4-[2-(5,5,8,8-tétraméthyl-3-propoxy-5,6,7,8-tétrahydronaphtalène-2-yl)vinyl]benzoïque,
- acide (E)-4-[2-(5,5,8,8-tétraméthyl-3-propoxy-5,6,7,8-tétrahydronaphtalène-2-yl)vinyl]benzoïque,
- acide (E)-4-[2-(5,5,8,8-tétraméthyl-3-pentoxy-5,6,7,8-tétrahydronaphtalène-2-yl)vinyl]benzoïque,
- ester éthylique de l'acide (2E,4E,6E)-3-méthyl-7-(5,5,8,8-tétraméthyl-3-propoxy-5,6,7,8-tétrahydro-naphtalène-2-yl)-hepta-2,4,6-triènecarboxylique,
- acide (2E,4E,6E)-3-méthyl-7-(5,5,8,8-tétraméthyl-3-propoxy-5,6,7,8-tétrahydronaphtalène-2-yl)-hepta-2,4,6-triènecarboxylique,
- acide (2Z,4E,6E)-3-méthyl-7-(5,5,8,8-tétraméthyl-3-propoxy-5,6,7,8-tétrahydronaphtalène-2-yl)-hepta-2,4,6-triènecarboxylique,
- acide (2E,4E,6E)-3-méthyl-7-(5,5,8,8-tétraméthyl-3-pentoxy-5,6,7,8-tétrahydronaphtalène-2-yl)-hepta-2,4,6-triènecarboxylique,
- acide (2Z,4E,6E)-3-méthyl-7-(5,5,8,8-tétraméthyl-3-pentoxy-5,6,7,8-tétrahydronaphtalène-2-yl)-hepta-2,4,6-triènecarboxylique,
- ester éthylique de l'acide (E)-4-[2-(7,7-diméthyl-3-octyl-6,7,8,9-tétrahydro-5H-benzocycloheptène-2-yl)-vinyl]benzoïque,

- acide (E)-4-[2-(7,7-diméthyl-3-octyl-6,7,8,9-tétrahydro-5H-benzocyclo-heptène-2-yl)vinyl]benzoïque,
- acide rac-(E)-4-[2-(5,7,7-triméthyl-3-octyl-6,7,8,9-tétrahydro-5H-benzocycloheptène-2-yl)vinyl]benzoïque,
- acide (E)-4-[2-(3-butyl-5,5-diméthyl-6,7,8,9-tétrahydro-5H-benzocycloheptène-2-yl)-vinyl]benzoïque,
- acide (E)-4-[2-(3-hexyl-7,7-diméthylindane-2-yl)-vinyl]benzoïque,
- acide (E)-4-[2-(3-hexyl-5,5-diméthyl-6,7,8,9-tétrahydro-5H-benzocycloheptène-2-yl)-vinyl]benzoïque,
- acide (E)-4-[2-(3-butyl-9,9-diméthyl-6,7,8,9-tétrahydro-5H-benzocycloheptène-2-yl)-vinyl]benzoïque,
- ester éthylique de l'acide (E)-4-[2-[(5RS,9SR,10RS)-3-heptyloxy-10-hydroxy-5,9-diméthyl-6,7,8,9-tétrahydro-5,9-méthano-5H-benzocycloheptène-2-yl]vinyl]benzoïque,
- acide (E)-4-[2-[(5RS,9SR,10RS)-3-heptyloxy-10-hydroxy-5,9-diméthyl-6,7, 8,9-tétrahydro-5,9-méthano-5H-benzocycloheptène-2-yl]vinyl]benzoïque,
- ester éthylique de l'acide (E)-4-[2-[(5RS,9SR,10RS)-3-butoxy-10-hydroxy-5,9-diméthyl-6,7,8,9-tétrahydro-5,9-méthano-5H-benzocycloheptène-2-yl]vinyl]benzoïque,
- acide (E)-4-[2-[(5RS,9SR,10RS)-3-butoxy-10-hydroxy-5,9-diméthyl-6,7,8,9-tétrahydro-5,9-méthano-5H-benzo-cycloheptène-2-yl]vinyl]benzoïque.

**10.** Utilisation selon la revendication 1 des composés suivants :

- ester éthylique de l'acide (2E,4E)-3-méthyl-7-(5,5,8,8-tétraméthyl-3-pentyl-5,6,7,8-tétrahydro-naphtalène-2-yl)-hepta-2,4-diène-6-ynoïque,
- acide (2E,4E)-3-méthyl-7-(5,5,8,8-tétraméthyl-3-pentyl-5,6,7,8-tétrahydronaphtalène-2-yl)-hepta-2,4-diène-6-ynoïque,
- acide (2E,4E)-3-méthyl-7-(5,5,8,8-tétraméthyl-3-pentyl-5,6,7,8-tétrahydronaphtalène-2-yl)-hepta-2,4-diène-6-ynecarboxylique,
- ester éthylique de l'acide (2E,4E,6E)-(5RS,9SR,10RS)-7-(3-butoxy-10-tert-butyldiméthylsilyloxy-5,9-diméthyl-6,7,8,9-tétrahydro-5,9-méthano-5H-benzocycloheptène-2-yl)-3-méthyl-hepta-2,4,6-triénoïque,
- ester éthylique de l'acide (2E,4E,6E)-(5RS,9SR,10RS)-7-(3-butoxy-10-hydroxy-5,9-diméthyl-6,7,8,9-tétrahydro-5,9-méthano-5H-benzocycloheptène-2-yl)-3-méthyl-hepta-2,4,6-triénoïque,
- acide (2E,4E,6E)-(5RS,9SR,10RS)-7-(3-butoxy-10-hydroxy-5,9-diméthyl-6,7,8,9-tétrahydro-5,9-méthano-5H-benzocycloheptène-2-yl)-3-méthyl-hepta-2,4,6-triénoïque.

**11.** Utilisation selon la revendication 1 du composé suivant :

- acide (E)-4-[2-(3-hexanoyl-5,5,8,8-tétraméthyl-5,6,7,8-tétrahydronaphtalène-2-yl)vinyl]benzoïque.

**12.** Utilisation selon la revendication 1 des composés suivants :

- 2-bromo-3-propargyloxy-5,5,8,8-tétraméthyl-5,6,7,8-tétrahydro-2-naphtalène,
- 2-formyl-3-pent-2-ynyloxy-5,5,8,8-tétraméthyl-5,6,7,8-tétrahydronaphtalène,
- ester éthylique de l'acide (E)-4-[2-(3-pent-2-ynyloxy-5,5,8,8-tétraméthyl-5,6,7,8-tétrahydronaphtalène-2-yl)-vinyl]benzoïque,
- acide (E)-4-[2-(3-pent-2-ynyloxy-5,5,8,8-tétraméthyl-5,6,7,8-tétrahydronaphtalène-2-yl)-vinyl]benzoïque,
- ester éthylique de l'acide (2E,4E,6E)-3-méthyl-7-(3-pent-2-ynyloxy-5,5,8,8-tétraméthyl-5,6,7,8-tétrahydro-naphtalène-2-yl)-hepta-2,4,6-triénoïque,
- acide (2E,4E,6E)-3-méthyl-7-(3-pent-2-ynyloxy-5,5,8,8-tétraméthyl-5,6,7,8-tétrahydronaphtalène-2-yl)-hepta-2,4,6-triénoïque,
- acide (E)-4-[2-(3-allyloxy-5,5,8,8-tétraméthyl-5,6,7,8-tétrahydronaphtalène-2-yl)vinyl]benzoïque,
- ester 4-[(E)-2-((E)-3-but-2-ényloxy-5,5,8,8-tétraméthyl-5,6,7,8-tétrahydronaphtalène-2-yl)-vinyl]-benzoïque,
- acide (2E,4E,6E)-7-(3-allyloxy-5,5,8,8-tétraméthyl-5,6,7,8-tétrahydronaphtalène-2-yl)-3-méthyl-hepta-2,4,6-triénoïque,
- acide (2E,4E,6E)-7-((E)3-but-2-ényloxy-5,5,8,8-tétraméthyl-5,6,7,8-tétrahydronaphtalène-2-yl)-3-méthyl-hepta-2,4,6-triénoïque.

**13.** Utilisation selon la revendication 1 de composés de formule I dans laquelle $R^2$ est un radical alkyle en $C_{2-8}$ ou alcoxy en $C_{1-7}$.

**14.** Composés de formule I selon la revendication 1 dans laquelle $R^2$ est un radical n-alcanoyle en $C_{2-8}$.

**15.** Composés de formule I selon la revendication 1 dans laquelle $R^2$ est - $OCH_2R^3$ et $R^3$ est un radical alcényle en $C_{2-}$

$_6$ ou alcynyle en $C_{2-6}$.